# EUROPÄISCHE PATENTSCHRIFT

(11) **EP 1 509 498 B1**
(45) Veröffentlichungstag und Bekanntmachung des Hinweises auf die Patenterteilung: **25.10.2006**
(21) Anmeldenummer: 03752723.1
(22) Anmeldetag: 05.05.2003
(51) Int. Cl.: C07C 381/00, A61P 9/06, A61P 9/10, A61K 31/155

(54) **PENTAFLUORSULFANYL-BENZOYLGUANIDINE, VERFAHREN ZU IHRER HERTELLUNG, IHRE VERWENDUNG ALS MEDIKAMENT ODER DIAGNOSTIKUM SOWIE SIE ENTHALTENDES MEDIKAMENT**
PENTAFLUOROSULFANYL-BENZOYLGUANIDINE, METHOD FOR THE PRODUCTION THEREOF AND ITS UTILIZATION AS MEDICAMENT OR DIAGNOSTIC AGENT AND MEDICAMENT CONTAINING SAME
PENTAFLUORSULFANYL-BENZOYL-GUANIDINES, PROCEDE POUR LES PREPARER, LEUR UTILISATION COMME MEDICAMENT OU AGENT DE DIAGNOSTIC, ET MEDICAMENT LES CONTENANT

(30) Priorität: 18.05.2002 DE 10222192
(43) Veröffentlichungstag der Anmeldung: 02.03.2005
(73) Patentinhaber: Sanofi-Aventis Deutschland GmbH, 65929 Frankfurt am Main (DE)
(72) Erfinder: KLEEMANN, Heinz-Werner, 65474 Bischofsheim (DE)
(86) Internationale Anmeldenummer: PCT/EP2003/004669
(87) Internationale Veröffentlichungsnummer: WO 2003/097591

(56) Entgegenhaltungen:
- EP-A- 0 686 627
- EP-A- 0 743 301
- EP-A- 0 754 680
- SHEPPARD W A: "ARYLSULFUR PENTAFLUORIDES" JOURNAL OF THE AMERICAN CHEMICAL SOCIETY, AMERICAN CHEMICAL SOCIETY, WASHINGTON, DC, US, Bd. 84, Nr. 16, 20. August 1962 (1962-08-20), Seiten 3064-3072, XP002073787 ISSN: 0002-7863

## Beschreibung

Die Erfindung betrifft Pentafluorsulfanyl-benzoylguanidine der Formel I und II 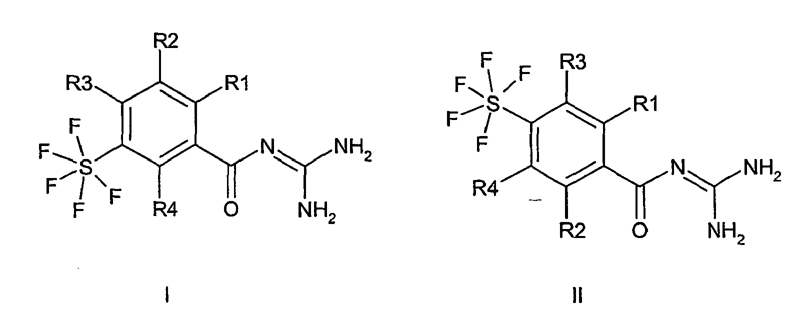 worin bedeuten
- R1: Wasserstoff, Alkyl mit 1, 2, 3 oder 4 C-Atomen, Alkoxy mit 1, 2, 3 oder 4 C-Atomen, F, Cl, Br, I, CN, NR10R11, -Oₚ-(CH₂)ₙ-(CF₂)ₒ-CF₃ oder -(SOₘ)_{q}-(CH₂)ᵣ(CF₂)ₛ-CF₃;
R10 und R11 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
- R2: Wasserstoff, F, Cl, Br, I, -CN, -SO₂CH₃, -(SOₕ)_{z}-(CH₂)ₖ -(CF₂)₁ -CF,₃, Alkyl mit 1, 2, 3, 4, 5 oder 6 C-Atomen, Cycloalkyl mit 3, 4, 5, 6, 7 oder 8 C-Atomen,
in dem 1, 2, 3 oder 4 Wasserstoff-Atome durch Fluoratome ersetzt sein können;
h Null, 1 oder 2;
z Null oder 1;
k Null, 1, 2, 3 oder 4;
l Null oder 1;
oder
- R2: -(CH₂)ₜ-Phenyl oder -O-Phenyl,
das unsubstituiert ist oder substituiert ist mit 1, 2 oder 3 Resten ausgewählt aus der Gruppe bestehend aus F, Cl, Br, I, -Oᵤ-(CH₂)ᵥ-CF₃, Alkoxy mit 1, 2, 3 oder 4 C-Atomen, Alkyl mit 1, 2, 3 oder 4 C-Atomen und -SO₂CH₃;
t Null, 1, 2, 3 oder 4;
u Null oder 1;
v Null, 1, 2 oder 3;
oder
- R2: -(CH₂)_{w}-Heteroaryl,
das unsubstituiert ist oder substituiert ist mit 1, 2 oder 3 Resten ausgewählt aus der Gruppe bestehend aus F, Cl, Br, I, -Oₓ-(CH₂)_{y}-CF₃, Alkoxy mit 1, 2, 3 oder 4 C-Atomen und Alkyl mit 1, 2, 3 oder 4 C-Atomen, -SO₂CH₃;
w Null, 1, 2, 3 oder 4;
x Null oder 1;
y Null, 1, 2 oder 3;
- R3 und R4: unabhängig voneinander Wasserstoff oder F; sowie deren pharmazeutisch verträgliche Salze.

Bevorzugt sind Verbindungen der Formel I und II, in denen bedeuten:
- R1: Wasserstoff, Alkyl mit 1, 2, 3 oder 4 C-Atomen, Alkoxy mit 1, 2, 3 oder 4 C-Atomen, F, Cl, NR10R11, -O-CH₂-CF₃ oder SOₘ(CH₂)ᵣCF₃;
R10 und R11 unabhängig voneinander Wasserstoff, Alkyl mit 1, 2, 3 oder 4 C-Atomen oder ―CH₂-CF₃;
m Null, 1 oder 2;
r Null oder 1;
- R2: Wasserstoff, F, Cl, -SO₂CH₃, -(SOₕ)_{z}-(CH₂)ₖ-CF₃, Alkyl mit 1, 2, 3 oder 4 C-Atomen, Cycloalkyl mit 3, 4, 5, 6 oder 7 C-Atomen,
in dem 1, 2, 3 oder 4 Wasserstoff-Atome durch Fluoratome ersetzt sein können;
h Null, 1 oder 2;
z Null oder 1;
k Null, 1, 2, 3 oder 4;
oder
- R2: Phenyl oder -O-Phenyl,
das unsubstituiert ist oder substituiert ist mit 1 oder 2 Resten ausgewählt aus der Gruppe bestehend aus F, Cl, -Oᵤ-(CH₂)ᵥ-CF₃, Methoxy, Ethoxy, Alkyl mit 1, 2, 3 oder 4 C-Atomen und -SO₂CH₃;
u Null oder 1;
v Null, 1, 2 oder 3;
oder
- R2: Heteroaryl,
das unsubstituiert ist oder substituiert ist mit 1 oder 2 Resten ausgewählt aus der Gruppe bestehend aus F, Cl, -Oₓ-(CH₂)_{y}-CF₃, Methoxy, Ethoxy, Alkyl mit 1, 2, 3 oder 4 C-Atomen und -SO₂CH₃;
x Null oder 1;
y Null, 1, 2 oder 3;
- R3 und R4: unabhängig voneinander Wasserstoff oder F;
sowie deren pharmazeutisch verträgliche Salze.

Besonders bevorzugt sind Verbindungen der Formel I und II, in denen bedeuten:
- R1: Wasserstoff, Alkyl mit 1, 2, 3 oder 4 C-Atomen, Methoxy, Ethoxy, F, Cl, NR10R11, -O-CH₂-CF₃ oder -SOₘ-(CH₂)ᵣ-CF₃;
R10 und R11 unabhängig voneinander Wasserstoff, Alkyl mit 1, 2, 3 oder 4 C-Atomen oder -CH₂-CF₃;
m Null, 1 oder 2;
r Null oder 1;
- R2: Wasserstoff, F, Cl, -SO₂CH₃, -(SOₕ)_{z}-(CH₂)ₖ-CF₃, Alkyl mit 1, 2, 3 oder 4 C-Atomen, Cycloalkyl mit 3, 4, 5, 6 oder 7 C-Atomen,
in dem 1, 2, 3 oder 4 Wasserstoff-Atome durch Fluoratome ersetzt sein können;
h Null oder 2;
z Null oder 1;
k Null oder 1;
oder
- R2: Phenyl oder -O-Phenyl,
das unsubstituiert ist oder substituiert ist mit 1 oder 2 Resten ausgewählt aus der Gruppe bestehend aus F, Cl, -O-(CH₂)ᵥ-CF₃, Methoxy, Ethoxy, Alkyl mit 1, 2, 3 oder 4 C-Atomen und -SO₂CH₃;
v Null, 1, 2 oder 3;
oder
- R2: Heteroaryl,
das unsubstituiert ist oder substituiert ist mit 1 oder 2 Resten ausgewählt aus der Gruppe bestehend aus F, Cl, -O-(CH₂)y-CF₃, Methoxy, Ethoxy, Alkyl mit 1, 2, 3 oder 4 C-Atomen und -SO₂CH₃;
y Null, 1, 2 oder 3;
- R3 und R4: Wasserstoff;
sowie deren pharmazeutisch verträgliche Salze.

Speziell bevorzugt ist es, wenn in den Verbindungen der Formel I und/oder II R1 für Wasserstoff, Alkyl mit 1, 2, 3 oder 4 C-Atomen, F oder Cl steht. Speziell bevorzugt ist es auch, wenn in den Verbindungen der Formel I und/oder II R2 für Wasserstoff, Alkyl mit 1, 2, 3 oder 4 C-Atomen, F, Cl oder -O-Phenyl, das unsubstituierte oder wie angegeben substituiert ist, steht.

Enthalten die Substituenten R1 bis R4 ein oder mehrere Asymmetriezentren, so können diese unabhängig voneinander sowohl S als auch R konfiguriert sein. Die Verbindungen können als optische lsomere, als Diastereomere, als Racemate oder als Gemische derselben vorliegen.

Die vorliegende Erfindung umfasst alle tautomeren Formen der Verbindungen der Formel I und II.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl und Hexyl. Bevorzugte Alkylreste sind Methyl, Ethyl, n-Propyl und Isopropyl. Substituierte Alkylreste können in beliebigen Positionen substituiert sein.

Beispiele für Cycloalkylreste sind Cyclopropyl, Cyclobutyl, Cyclopentyl, Cyclohexyl Cycloheptyl oder Cyclooctyl.

Substituierte Cycloalkylreste können in beliebigen Positionen substituiert sein.

Phenylreste können unsubstituiert sein oder einfach oder mehrfach, zum Beispiel einfach, zweifach oder dreifach, durch gleiche oder verschiedene Reste substituiert sein. Wenn ein Phenylrest substituiert ist, trägt er bevorzugt einen oder zwei gleiche oder verschiedene Substituenten. Dies gilt ebenso für substituierte Phenylreste in Gruppen wie zum Beispiel Phenylalkyl oder Phenyloxy. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden.

Heteroarylreste sind aromatische Ringverbindungen, in denen ein oder mehrere Ringatome Sauerstoffatome, Schwefelatome oder Stickstoffatome sind, z. B. 1, 2 oder 3 Stickstoffatome, 1 oder 2 Sauerstoffatome, 1 oder 2 Schwefelatome oder eine Kombinationen aus verschiedenen Heteroatomen. Die Heteroarylreste können über alle Positionen angebunden sein, zum Beispiel über 1-Position, 2-Position, 3-Position, 4-Position, 5-Position, 6-Position, 7-Position oder 8-Position. Heteroarylreste können unsubstituiert sein oder einfach oder mehrfach, zum Beispiel einfach, zweifach oder dreifach, durch gleiche oder verschiedene Reste substituiert sein. Dies gilt ebenso für die Heteroarylreste wie zum Beispiel im Rest Heteroarylalkyl. Heteroaryl bedeutet zum Beispiel Furanyl, Thienyl, Pyrrolyl, Imidazolyl, Pyrazolyl, Triazolyl, Tetrazolyl, Oxazolyl, lsoxazolyl, Thiazolyl, Isothiazolyl, Pyridyl, Pyrazinyl, Pyrimidinyl, Pyridazinyl, Indolyl, Indazolyl, Chinolyl, lsochinolyl, Phthalazinyl, Chinoxalinyl, Chinazolinyl und Cinnolinyl.

Als Heteroarylreste gelten insbesondere 2- oder 3-Thienyl, 2- oder 3-Furyl, 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder -5-yl, 1,2,4-Triazol-1-, -3- oder -5-yl, 1- oder 5-Tetrazolyl, 2-, 4- oder 5-Oxazolyl, 3-, 4- oder 5-lsoxazolyl, 1,2,3-Oxadiazol-4- oder -5-yl, 1,2,4-Oxadiazol-3- oder -5-yl, 1,3,4-Oxadiazol-2-yl oder -5-yl, 2-, 4- oder 5-Thiazolyl, 3-, 4- oder 5-lsothiazolyl, 1,3,4-Thiadiazol-2- oder -5-yl, 1,2,4-Thiadiazol-3- oder -5-yl, 1,2,3-Thiadiazol-4- oder -5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-lndolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also zum Beispiel 1-Oxy-2-, 3- oder 4-pyridyl.

Besonders bevorzugt sind die Heteroaromaten 2- oder 3-Thienyl, 2- oder 3-Furyl, 1-, 2- oder 3- Pyrrolyl, 1-, 2-, 4- oder 5-lmidazolyl, 2-, 3-, 4-, 5-, 6-, 7- oder 8-Chinolyl, 1-, 3-, 4- oder 5-Pyrazolyl, 2-, 3- oder 4-Pyridyl, 2- oder 3-Pyrazinyl, 2-, 4-, 5- oder 6-Pyrimidinyl und 3- oder 4-Pyridazinyl.

Die Erfindung betrifft weiterhin ein Verfahren zur Herstellung einer Verbindung der Formel I und II und/oder deren pharmazeutisch verträgliche Salze, dadurch gekennzeichnet, dass man eine Verbindung der Formel III oder IV 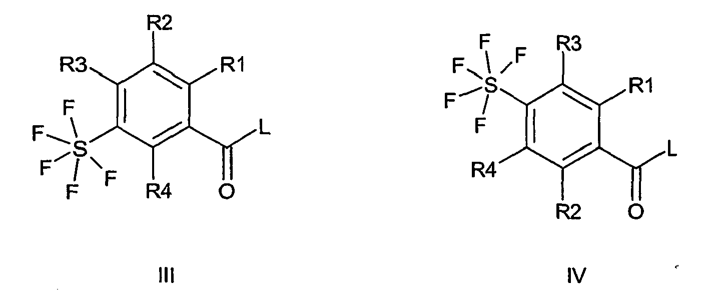 worin R1 bis R4 die angegebene Bedeutung besitzen und L für eine leicht nucleophil substituierbare Fluchtgruppe steht, mit Guanidin umsetzt.

Die aktivierten Säurederivate der Formel III und IV, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-lmidazolyl, bedeutet, erhält man vorteilhaft in an sich bekannter Weise aus den zugrundeliegenden Carbonsäurechloriden (Formel III, IV; L = Cl), die man ihrerseits wiederum in bekannter Weise aus den zugrundeliegenden Carbonsäuren (Formel III, IV; L = OH) beispielsweise mit Thionylchlorid herstellen kann.
Neben den Carbonsäurechloriden der Formel III und IV (L = Cl) lassen sich auch weitere aktivierte Säurederivate der Formel III und IV in an sich bekannter Weise direkt aus den zugrundeliegenden Benzoesäuren (Formel III, IV; L = OH) herstellen, wie die Methylester der Formel III und IV mit L = OCH₃ durch Behandeln mit gasförmigem HCl in Methanol, die Imidazolide der Formel III und IV durch Behandeln mit Carbonyldiimidazol [L = 1-Imidazolyl, Staab, Angew. Chem. Int. Ed. Engl. 1, 351 - 367 (1962)], die gemischten Anhydride der Formel III und IV mit Cl-COOC₂H₅ oder Tosylchlorid in Gegenwart von Triethylamin in einem inerten Lösungsmittel, wie auch Aktivierungen von Benzoesäuren mit Dicyclohexylcarbodümid (DCC) oder mit O-[(Cyano(ethoxycarbonyl)methylen)amino]-1,1,3,3-tetramethyluronium-tetrafluorborat ("TOTU") [Proceedings of the 21. European Peptide Symposium, Peptides 1990, Editors E. Giralt and D. Andreu, Escom, Leiden, 1991] möglich sind. Eine Reihe geeigneter Methoden zur Herstellung von aktivierten Carbonsäurederivaten der Formel III und IV sind unter Angabe von Quellenliteratur in J. March, Advanced Organic Chemistry, Third Edition (John Wiley & Sons, 1985, S. 350) angegeben.

Die Umsetzung eines aktivierten Carbonsäurederivates der Formel III und IV mit Guanidin erfolgt vorzugsweise in an sich bekannter Weise in einem protischen oder aprotischen polaren aber inerten organischen Lösungsmittel. Dabei haben sich bei der Umsetzung der Benzoesäuremethylester (III, IV; L = OCH₃) mit Guanidin Methanol, Isopropanol oder THF von 20°C bis zur Siedetemperatur dieser Lösungsmittel bewährt. Bei den meisten Umsetzungen von Verbindungen III und IV mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von III und IV mit Guanidin verwendet werden.

Wenn L die Bedeutung Cl hat, arbeitet man vorteilhaft unter Zusatz eines Säurefängers, zum Beispiel in Form von überschüssigen Guanidin zur Abbindung der Halogenwasserstoffsäure.

Zur Erfindung gehören auch Vorprodukte der Formel V 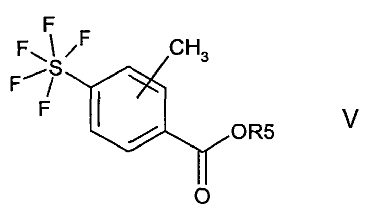 mit R5 gleich Wasserstoff oder (C₁-C₄)- Alkyl, in denen die Methylgruppe in der 2-Position oder in der 3-Position des aromatischen Ringes stehen kann.

Pentafluorsulfanyl-benzoylguanidine I und II sind im allgemeinen schwache Basen und können Säure unter Bildung von Salzen binden. Als Säureadditionssalze kommen Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I und II sind substituierte Acylguanidine und inhibieren den zellulären Natrium-Protonen-Antiporter (Na⁺/H⁺-Exchanger, NHE).

Gegenüber den bekannten Verbindungen zeichnen sich die erfindungsgemäßen Verbindungen durch eine außerordentlich hohe Wirksamkeit in der Inhibition des Na⁺/H⁺-Austauschs aus, sowie durch verbesserte ADMET-Eigenschaften aus. Durch die xenobiotische Struktur (insbesondere durch die Einführung der eher "unnatürlichen/naturfremden" SF₅-Substituenten) wird die metabolische Angriffsmöglichkeit erschwert. Das führt u.a. zu längeren S9-Stabilitäten (Leberstabilitäten, Stabilität gegenüber enzymatischem Angriff) und einer längeren Halbwertszeit in vivo. Dabei wird das Resorptionsverhalten nicht signifikant beeinflusst und die hohe Bioverfügbarkeit der Acylguanidine bleibt erhalten.

Im Gegensatz zu einigen in der Literatur beschriebenen Acylguanidinen zeigen die hier beschriebenen Verbindungen der Formel I und/oder II und/oder deren pharmazeutisch verträgliche Salze keine unerwünschten und nachteiligen saliduretischen Eigenschaften.

Aufgrund der NHE-inhibitorischen Eigenschaften eignen sich die Verbindungen der Formel I und/oder II und/oder deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.

Da NHE Inhibitoren in überwiegender Weise über ihre Beeinflussung der zellulären pH-Regulation wirken, können diese generell in günstiger Weise mit anderen, den intrazellulären pH-Wert regulierenden Verbindungen kombiniert werden, wobei Inhibitoren der Enzymgruppe der Carboanhydrasen, Inhibitoren der Bicarbonationen transportierenden Systeme wie des Natrium-Bicarbonat-Cotransporters (NBC) oder des Natrium abhängigen Chlorid-Bicarbonat-Austauschers (NCBE), sowie NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.

So eignen sich die erfindungsgemäßen Inhibitoren des NHE zur Behandlung von Krankheiten, die durch lschämie und durch Reperfusion hervorgerufen werden.

Die hier beschriebenen Verbindungen sind infolge ihrer pharmakologischen Eigenschaften als antiarrhythmische Arzneimittel geeignet.
Durch ihre cardioprotektive Komponente eignen sich die NHE-Inhibitoren der Formel I und/oder II und/oder deren pharmazeutisch verträgliche Salze hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I und/oder II und/oder deren pharmazeutisch verträgliche Salze infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die erfindungsgemäßen Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Es zeigte sich, dass die erfindungsgemäßen Verbindungen außerordentlich wirksame Arzneimittel sind gegen lebensbedrohliche Arrhythmien. Kammerflimmern wird beendet und der physiologische Sinus-Rhythmus des Herzens wiederhergestellt.

Da NHE1-Inhibitoren menschliches Gewebe und Organe, insbesondere das Herz, nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist die kombinierte Verabreichung mit Verbindungen der Formel I und/oder II und/oder deren pharmazeutisch verträgliche Salze geeignet, die cytotoxischen, insbesondere cardiotoxischen Nebenwirkungen der genannten Verbindungen zu inhibieren. Durch die Verminderung der cytotoxischen Effekte, insbesondere der Cardiotoxizität, infolge Ko-Medikation mit NHE1-Inhibitoren kann außerdem die Dosis der cytotoxischen Therapeutika erhöht und/oder die Medikation mit solchen Arzneimitteln verlängert werden. Der therapeutische Nutzen einer solchen cytotoxischen Therapie kann durch die Kombination mit NHE-Inhibitoren erheblich gesteigert werden.

Außerdem können die erfindungsgemäßen NHE1-Inhibitoren der Formel I und/oder II und/oder deren pharmazeutisch verträgliche Salze bei einer herzschädigenden Überproduktion von Schilddrüsenhormonen, der Thyreotoxikose, oder bei der externen Zufuhr von Schilddrüsenhormonen Verwendung finden. Die Verbindungen der Formel I und/oder II und/oder deren pharmazeutisch verträgliche Salze eignen sich somit zur Verbesserung der Therapie mit cardiotoxischen Arzneimitteln.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie zum Beispiel zur Behandlung des Schlaganfalls oder des Hirnödems geeignet sind.

Die Verbindungen der Formel I und/oder II und/oder deren pharmazeutisch verträgliche Salze eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychische Depressionszustände, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydrasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäß verwendeten Verbindungen der Formel I und/oder II und/oder deren pharmazeutisch verträgliche Salze ebenfalls zur Behandlung von Formen des Schocks, wie beispielweise des allergischen, cardiogenen, hypovolämischen und des bakteriellen Schocks.

Die Verbindungen der Formel I und/oder II und/oder deren pharmazeutisch verträgliche Salze können ebenfalls zur Prävention und zur Behandlung thrombotischer Erkrankungen verwendet werden, da sie als NHE-Inhibitoren sowohl die Plättchenaggregation selbst inhibieren können. Darüberhinaus können sie die nach Ischämie und Reperfusion stattfindende überschießende Freisetzung von Entzündungs- und Gerinnungsmediatoren, insbesondere des *von Willebrand Faktors* und der thrombogenen Selectin-Proteine hemmen bzw. verhindern. Damit kann die pathogene Wirkung bedeutender thrombogener Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan RezeptorAntagonisten, Phosphodiesterase-Inhibitoren, Factor-Vlla-Antagonisten, Clopidogrel, Ticolopidin usw. Eine kombinierte Anwendung der vorliegenden NHE-Inhibitoren mit NCBE-Inhibitoren und/oder mit Inhibitoren der Carboanhydrase, wie beispielsweise mit Acetazolamid, ist besonders günstig.

Darüber hinaus zeichnen sich die erfindungsgemäß verwendeten Verbindungen der Formel I und/oder II und/oder deren pharmazeutisch verträgliche Salze durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I und/oder II und/oder deren pharmazeutisch verträgliche Salze als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden.

Es konnte gezeigt werden, dass durch die erfindungsgemäßen Verbindungen die Zellmigration inhibiert wird. Daher kommen die Verbindungen der Formel I und/oder II und/oder deren pharmazeutisch verträgliche Salze als wertvolle Therapeutika für Krankheiten in Frage, bei denen die Zellmigration eine primäre oder sekundäre Ursache darstellt, wie beispielsweise Krebserkrankungen mit ausgeprägter Neigung zur Metastasierung.

Die Verbindungen der Formel I und/oder II und/oder deren pharmazeutisch verträgliche Salze zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.
Sie können somit zur Behandlung von Organhypertrophien und -hyperplasien, beispielsweise des Herzens und der Prostata verwendet werden. Sie sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

Da der NHE bei essentiellen Hypertonikern signifikant erhöht ist, eignen sich die Verbindungen der Formel I und/oder II und/oder deren pharmazeutisch verträgliche Salze zur Prävention und zur Behandlung des Bluthochdrucks und von Herz-Kreislauferkrankungen.
Dabei können sie allein oder mit einem geeigneten Kombinations- und Formulierungspartner zur Bluthochdruckbehandlung und von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amiloride, Triamteren, Spironolacton oder Epleron, kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, albuterol usw., Antagonisten des Angiotensin-Rezeptors und seiner Rezeptor-Subtypen wie Losartan, lrbesartan, Valsartan, Omapatrilat, Gemopatrilat, Endothelin-Antagonisten, Reninlnhibitoren, Adenosin- Rezeptoragonisten, Inhibitoren und Aktivatoren von Kaliumkanälen wie Glibenclamide, Glimepirid, Diazoxide, Cromokalim, Minoxidil und deren Derivate, Aktivatoren des mitochondrialen ATP-sensitiven Kaliumkanals (mitoK(ATP) Kanal), Inhibitoren des Kv1.5 usw.

Es zeigte sich, dass NHE1 Inhibitoren der Formel I und/oder II und/oder deren pharmazeutisch verträgliche Salze eine signifikante antiphlogistische Wirkung haben und somit als Antünflammatorika verwendet werden können. Dabei fällt die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet.

Es wurde außerdem gefunden, dass Verbindungen der der Formel I und/oder II und/oder deren pharmazeutisch verträgliche Salze eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, dass NHE1 Inhibitoren bezüglich der Beeinflussung der Serumlipidspiegel wertvolle therapeutisch verwertbare Eigenschaften zeigen. So erniedrigen sie signifikant die erhöhte Serum Konzentrationen von LDL und VLDL, wie sie beispielweise durch erhöhte diätetische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie zum Beispiel beim Diabetes vorkommen. Darüber hinaus führen die Verbindungen der Formel I und/oder II und/oder deren pharmazeutisch verträgliche Salze zu einer deutlichen Reduktion der durch Stoffwechselanomalien induzierten Infarkte und insbesondere zu einer signifikanten Verminderung der induzierten Infarktgröße und dessen Schweregrades. Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I und/oder II und/oder deren pharmazeutisch verträgliche Salze mit einem blutfettspiegelsenkenden Wirkstoff, bevorzugt mit einem HMG-CoA-Reduktaseinhibitor (zum Beispiel Lovastatin oder Pravastatin), wobei letzterer eine hypolipidämische Wirkung herbeiführt und dadurch die hypolipidämischen Eigenschaften des NHE-Inhibitors der Formel I und/oder II und/oder deren pharmazeutisch verträgliche Salze steigert, erweist sich als günstige Kombination mit verstärkter Wirkung und vermindertem Wirkstoffeinsatz.
So führen Verbindungen der Formel I und/oder II und/oder deren pharmazeutisch verträgliche Salze zu einen wirksamen Schutz gegen Endothelschädigungen unterschiedlicher Genese. Mit diesem Schutz der Gefäße gegen das Syndrom der endothelialen Dysfunktion sind Verbindungen der Formel I und/oder II und/oder deren pharmazeutisch verträgliche Salze wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem wurde gefunden, dass Benzoylguanidine der Formel I und/oder II und/oder deren pharmazeutisch verträgliche Salze geeignet in der Behandlung des nichtinsulinabhängigen Diabetes (NIDDM) sind, wobei die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburide, Glimepiride, Tolbutamid usw., einem Glucosidase Inhibitor, einem PPAR Agonisten, wie Rosiglitazone, Pioglitazone etc., mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken die Verbindungen der Formel I und/oder II und/oder deren pharmazeutisch verträgliche Salze der Entstehung diabetischer Spätkomplikationen entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den soeben unter NIDDM-Behandlung beschriebenen antidiabetischen Arzneimitteln vorteilhaft kombiniert werden. Der Kombination mit einer günstigen Darreichungsform von Insulin dürfte dabei eine besondere Bedeutung zukommen.

Die erfindungsgemäßen NHE-Inhibitoren der Formel I und/oder II und/oder deren pharmazeutisch verträgliche Salze zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die unabhängig von akuten Mangeldurchblutungszuständen sind und bei normalen, nicht-ischämischen Bedingungen auftreten. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im altemden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. Die Verbindungen der Formel I und/oder II und/oder deren pharmazeutisch verträgliche Salze eignen sich somit hervorragend zur Behandlung und Prävention der altersbedingt fortschreitenden endothelialen Dysfunktion, insbesondere von claudicatio intermittens.

Beispiel einer weiteren den Altersprozess charakterisierenden Messgröße ist die Abnahme der Kontraktilität des Herzens und die Abnahme der Anpassung des Herzens an eine geforderte Pumpleistung des Herzens. Diese verminderte Herzleistungsfähigkeit als Folge des Alterungsprozesses ist in den meisten Fällen verbunden mit einer Dysfunktion des Herzens, die unter anderem durch eine Einlagerung von Bindegewebe ins Herzgewebe verursacht wird. Diese Bindegewebseinlagerung ist gekennzeichnet durch eine Zunahme des Herzgewichtes, durch eine Vergrößerung des Herzens und durch eine eingeschränkte Herzfunktion. Es war überraschend, dass eine derartige Alterung des Organs Herz nahezu komplett inhibiert werden konnte. Die Verbindungen der Formel I und/oder II und/oder deren pharmazeutisch verträgliche Salze eignen sich somit hervorragend zur Behandlung und Prävention der Herzinsuffizienz, des congestive heart failure (CHF).

Während in den vorangegangenen Patenten und Patentanmeldungen die Behandlung von unterschiedlichen, bereits eingetretenen Formen von Krebserkrankungen beansprucht wurden, war es nun außerordentlich überraschend, dass nicht nur die bereits eingetretene Krebserkrankung durch Proliferationshemmung geheilt werden kann, sondern dass auch die altersbedingte Entstehungshäufigkeit von Krebs durch NHE-Inhibitoren verhindert und hochsignifikant verzögert wird. Besonders bemerkenswert ist der Befund, dass altersbedingt auftretenden Erkrankungen aller Organe und nicht nur bestimmter Krebsformen unterbunden bzw. hochsignifikant verzögert auftreten. Die Verbindungen der Formel I und/oder II und/oder deren pharmazeutisch verträgliche Salze eignen sich somit hervorragend zur Behandlung und insbesondere der Prävention von altersbedingten Formen von Krebs.

Es wird nun nicht nur eine über das statistische Normalmaß hinaus, zeitlich hochsignifikant verschobene Verzögerung des Eintretens altersbedingter Erkrankungen aller untersuchten Organe einschließlich Herz, Gefäße, Leber usw., sowie eine hochsignifikante Verzögerung von Alterskrebs festgestellt. Vielmehr kommt es auch überraschenderweise zu einer Lebensverlängerung in einem Ausmaß, das bislang durch keine andere Medikamentengruppe bzw. durch irgendwelche Naturstoffe erreicht werden konnte. Diese einzigartige Wirkung der NHE-Inhibitoren ermöglicht es auch, neben der alleinigen Wirkstoffanwendung an Mensch und Tier diese NHE-Inhibitoren mit anderen gerontologisch verwendeten Wirkprinzipien, Maßnahmen, Substanzen und Naturstoffen zu kombinieren, denen ein anderer Wirkmechanismus zugrunde liegt. Derartige in der gerontologischen Therapie verwendete Wirkstoffklassen sind: insbesondere Vitamine und antioxidativ wirksame Stoffe. Da eine Korrelation zwischen kalorischer Belastung bzw. Nahrungsaufnahme und Alterungsprozeß besteht, kann die Kombination mit diätetischen Maßnahmen zum Beispiel mit Appetitszüglern erfolgen. Ebenso kann eine Kombination mit blutdrucksenkenden Medikamenten, wie mit ACE-Hemmern, Angiotensinrezeptorantagonisten, Diuretika, Ca⁺²-Antagonisten etc. oder mit stoffwechselnormalisierenden Medikamenten, wie Cholesterinsenkern, gedacht werden.
Die Verbindungen der Formel I und/oder II und/oder deren pharmazeutisch verträgliche Salze eignen sich somit hervorragend zur Prävention altersbedingter Gewebsveränderungen und zur Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Beansprucht wird weiterhin ein Heilmittel für die humane, veterinäre oder phytoprotektive Anwendung enthaltend eine wirksame Menge einer Verbindung der Formel I und/oder II und/oder deren pharmazeutisch verträgliche Salze, zusammen mit pharmazeutisch annehmbaren Träger- und Zusatzstoffen, allein oder in Kombination mit anderen pharmakologischen Wirkstoffen oder Arzneimitteln.

Arzneimittel, die eine Verbindung der Formel I und/oder II und/oder deren pharmazeutisch verträgliche Salze enthalten, können dabei zum Beispiel oral, parenteral, intravenös, rektal, perkutan oder durch Inhalation appliziert werden, wobei die bevorzugte Applikation von dem jeweiligen Erscheinungsbild der Erkrankung abhängig ist. Die Verbindungen I und/oder II können dabei allein oder zusammen mit galenischen Hilfsstoffen zur Anwendung kommen, und zwar sowohl in der Veterinärals auch in der Humanmedizin. Die Arzneimittel enthalten Wirkstoffe der Formel I und/oder II und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können zum Beispiel Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, intramuskulären oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen zum Beispiel in Frage: Wasser, physiologische Kochsalzlösung oder Alkohole, zum Beispiel Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet zum Beispiel Lösungen, Suspensionen oder Emulsionen des Wirkstoffes der Formel I und/oder II und/oder deren pharmazeutisch verträgliche Salze in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und/oder II und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I und/oder II und/oder deren pharmazeutisch verträgliche Salze bei einem etwa 75 kg schweren Patienten mindestens 0,001 mg/kg, beispielsweise 0,01 mg/kg, bis höchstens 10 mg/kg, beispielsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, zum Beispiel bis zu 4 Einzeldosen pro Tag. Insbesondere bei i.v. Anwendung, etwa bei einem Infarktpatienten auf der Intensivstation können beispielsweise bis zu 700 mg pro Tag notwendig werden und können die erfindungsgemäßen Verbindungen durch Infusion verabreicht werden.

### Liste der Abkürzungen:

- ADMET: Absorption ― Distribution ― Metabolismus ― Ausscheidung - Toxikologie
- CDI: Di-imidazol-1-yl-methanon
- DIP: Diisopropylether
- DIPEA: Diisopropylethylamin
- DME: 1,2-Dimethoxyethan
- DMF: N,N-Dimethylformamid
- EE: Ethylacetat (EtOAc)
- eq.: Äquivalent
- HEP: n-Heptan
- HOAc: Essigsäure
- KOtBu: Kalium-2-methyl-propan-2-olat
- MeOH: Methanol
- mp: Schmelzpunkt
- MTB: tert.-Butyl-methylether
- Pd(dppf)₂: [1,1'-Bis-(diphenylphosphino)-ferrocen]palladium(II)chlorid-Methylenchlorid-Komplex (1:1)
- RT: Raumtemperatur
- TFA: Trifluoressigsäure
- THF: Tetrahydrofuran
- TMEDA: N,N,N',N'-Tetramethyl-ethan-1,2-diamin

### Experimenteller Teil

### Beispiel 1

### 3-Pentafluorsulfanyl-benzoylguanidin, Hydrochlorid

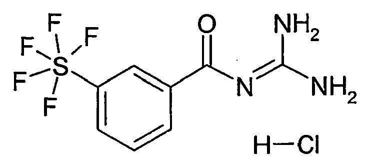

### a) 3-Pentafluorsulfanyl-benzoesäure

700 mg (3-lodphenyl)schwefelpentafluorid (Tetrahedron 56, (2000) 3399) und 300 mg Methyliodid wurden in 20 ml Diethylether (wasserfrei) gelöst und die Lösung zu 155 mg Magnesium/10 ml Diethylether langsam zugetropft. Eine Stunde wurde im Rückfluss nachgerührt, dann auf-10°C gekühlt und das Reaktionsgemisch mit CO₂ unter Normaldruck begast. 16 Stunden lang wurde bei RT gerührt, anschließend das Reaktionsgemisch mit verdünnter wässeriger HCl-Lösung auf pH = 1 gestellt und 3 mal mit je 50 ml EE extrahiert. Über MgSO₄ wurde getrocknet und das Solvens im Vakuum entfernt. Chromatographie an Kieselgel mit DIP/2% HOAc lieferte 200 mg eines farblosen, amorphen Pulvers.

| | |
|---|---|
| Rf (DIP/2%HOAc) = 0.51 | MS (ES⁺) : 249 |

### b) 3-Pentafluorsulfanyl-benzoylguanidin, Hydrochlorid

30 mg 3-Pentafluorsulfanyl-benzoesäure wurden zusammen mit 24 mg CDI in 5 ml DMF (wasserfrei) 3 Stunden lang bei RT gerührt. Daneben wurden 69 mg Guanidinium-chlorid zusammen mit 68 mg KOtBu in 5 ml DMF (wasserfrei) 30 Minuten lang bei RT gerührt. Die beiden Lösungen wurden anschließend vereinigt und 18 Stunden lang bei RT stehen gelassen. Das Reaktionsgemisch wurde mit 20 ml EE verdünnt und 3 mal mit je 5 ml einer halbkonzentrierten wässerigen NaHCO₃-Lösung gewaschen. Über MgSO₄ wurde getrocknet, das Solvens im Vakuum entfernt und mit überschüssiger 5% wässeriger HCl-Lösung aufgenommen. Die flüchtigen Bestandteile wurden im Vakuum entfernt und man erhielt 33 mg eines amorphen Feststoffs.

| | |
|---|---|
| Rf (EE) = 0.30 | MS (ES⁺) : 290 |

### Beispiel 2: 4-Pentafluorsulfanyl-benzoylguanidin, Hydrochlorid

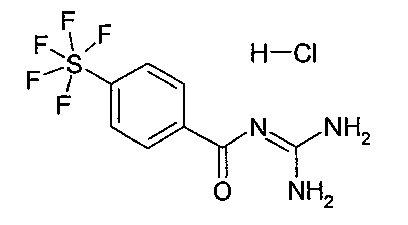

### a) 4-Pentafluorsulfanyl-benzoesäure

2.7 g (4-lodphenyl)schwefelpentafluorid (Tetrahedron 56, (2000) 3399) wurden analog Beispiel 1 a) umgesetzt und man erhielt 630 mg eines farblosen, amorphen Feststoffs.

| | |
|---|---|
| Rf (DIP/2%HOAc) = 0.51 | MS (ES⁺): 249 |

### b) 4-Pentafluorsulfanyl-benzoylguanidin, Hydrochlorid

50 mg 4-Pentafluorsulfanyl-Benzoesäure wurden analog Beispiel 1 b) umgesetzt und man erhielt 33 mg der Titelverbindung des Beispiels 2 als amorphes Pulver.

| | |
|---|---|
| Rf (EE) = 0.30 | MS (ES⁺) : 290 |

### Beispiel 3: 4-Pentafluorsulfanyl-2-methyl-benzoylguanidin

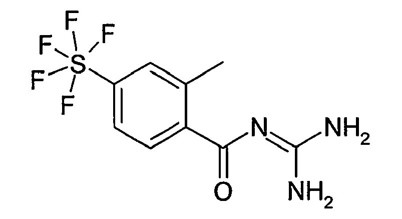

### a) 4-Pentafluorsulfanyl-2-methyl-benzoesäure

3.09 g TMEDA wurden in 30 ml THF (wasserfrei) gelöst und bei -90°C 20.5 ml einer 1.3 M Lösung von sec.-Butyllithium in Cyclohexan zugetropft. Anschließend wurde eine Lösung von 3.0 g 4-Pentafluorsulfanyl-benzoesäure in 20 ml THF (wasserfrei) bei -90°C zugetropft. Eine Stunde lang wurde bei -90°C nachgerührt, dann eine Lösung von 5.15 g Methyliodid in 20 ml THF (wasserfrei) zugetropft. Dabei wurde die Temperatur bei -80°C gehalten. Anschließend wurde 20 Minuten lang bei -78°C nachgerührt, 100 ml Wasser zugespritzt, mit einer verdünnten wäßrigen HCl-Lösung auf pH = 1 gestellt und 3 mal mit je 100 ml MTB extrahiert. Über MgSO₄ wurde getrocknet und das Solvens im Vakuum entfernt. Der Rückstand wurde zunächst chromatographiert an Kieselgel mit DIP/2%HOAc, und man erhielt 1.60 g eines Gemisches aus 4-Pentafluorsulfanyl-benzoesäure und 4-Pentafluorsulfanyl-2-methylbenzoesäure. Dieses Gemisch wurde unter den folgenden Bedingungen erneut chromatographiert:
- Säule:: Waters X-terra 250x50 mm + Vorsäule 50x50 mm
- Packung:: C18 10 µM
- Fluss:: 150 ml/min
Gradient (linearer Verlauf):
- Solvent A: Wasser + 2% Trifluoressigsäure
- Solvent B: Acetonitril

| Time [min] | Solv.A [%] | Solv.B [%] |
|---|---|---|
| 0.00 | 90 | 10 |
| 4.00 | 90 | 10 |
| 24.00 | 25 | 75 |
| 25.00 | 5 | 95 |
| 30.00 | 5 | 95 |
| 31.00 | 90 | 10 |
| 35.00 | 90 | 10 |

Man erhielt 900 mg der Titelverbindung in Form eines farblosen Feststoffs mit einer Retentionszeit von 21.14 Minuten neben 360 mg 4-Pentafluorsulfanyl-benzoesäure mit einer Retentionszeit von 20.18 Minuten (detektiert bei 220 nm Wellenlänge).

| | | |
|---|---|---|
| Rf (DIP/2%HOAc) = 0.5 | MS (CI⁺) : 263 ; | MS (ES⁻) : 261 |

### b) 4-Pentafluorsulfanyl-2-methyl-benzoylguanidin

910 mg 4-Pentafluorsulfanyl-2-methyl-benzoesäure wurden in 25 ml DMF (wasserfrei) gelöst, bei RT 844 mg CDI zugegeben und 6 Stunden bei RT gerührt (Lösung 1). Außerdem wurden 1.988 g Guanidinium-chlorid und 1.947 g KOtBu in 10 ml DMF (wasserfrei) bei RT 30 Minuten lang gerührt (Lösung 2). Anschließend wurde Lösung 2 zu Lösung 1 zugegeben und 17 Stunden bei RT gerührt. Das Reaktionsgemisch wurde dann mit 200 ml MTB verdünnt und 1 mal mit 100 ml Wasser gewaschen. Dieses Wasser wurde anschließend mit 100 ml MTB extrahiert. Die vereinigten organischen Phasen wurden dann nochmals 3 mal mit je 50 ml Wasser gewaschen und über MgSO₄ getrocknet. Das Solvens wurde im Vakuum entfernt und der Rückstand an Kieselgel mit EE chromatographiert. Man erhielt 600 mg weißer Kristalle, mp 185°C.

| | |
|---|---|
| Rf (EE) = 0.22 | MS (ES⁺) : 304 |

### Beispiel 4 N-[2-Chlor-4-pentafluorsulfanyl-benzoyl]-guanidin

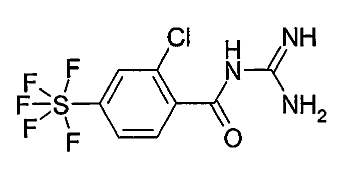

### a) 2-Chlor-4-pentafluorsulfanyl-benzoesäure

20.0 ml TMEDA wurden in 150 ml THF(wasserfrei) gelöst und bei einer Temperatur zwischen -80°C und -90°C mit 93.0 ml einer 1.25 M Lösung von sec. BuLi in Cyclohexan versetzt. Anschließend wurde bei einer Temperatur zwischen -87°C und - 93°C eine Lösung von 4-Pentafluorsulfanyl-benzoesäure (Beispiel 2a) in 50 ml THF(wasserfrei) innerhalb von 35 Minuten zugetropft. 2 Stunden wurde bei -90°C nachgerührt und anschließend 38.2 g 1,1,1,2,2,2-Hexachlor-ethan in 60 ml THF (wasserfrei) bei -90°C zugetropft. Man ließ auf-70°C erwärmen, dann wurden 100 ml Wasser zugetropft. Das Solvens wurde im Vakuum entfernt und der Rückstand an Kieselgel chromatographiert mit DIP/2%HOAc. Man erhielt 5.0 g des gewünschten Produktes als teilweise kristallisierendes Öl.

| | |
|---|---|
| R_{f} (DIP/2%HOAc) = 0.21 | MS (EI) : 282 (M+1)⁺ |

### b) N-[2-Chlor-4-pentafluorsulfanyl-benzoyl]-guanidin

170 mg 2-Chlor-4-pentafluorsulfanyl-benzoesäure wurden in 3 ml DMF (wasserfrei) gelöst und bei RT 127 mg CDI zugegeben. 2 Stunden wurde bei RT nachgerührt und man erhielt das intermediäre Imidazolid.
Daneben wurden 337 mg KOt-Bu in 5 ml DMF (wasserfrei) gelöst und eine Lösung von 344 mg Guanidin-Hydrochlorid in 5 ml DMF (wasserfrei) zugetropft. Die Lösung wurde 30 Minuten bei RT nachgerührt und anschließend die Lösung des Imidazolids bei RT zugetropft. Das Reaktionsgemisch wurde 16 Stunden bei RT stehen gelassen und dann das Solvens imVakuum entfernt. Der Rückstand wurde in 50 ml EE/20 ml Wasser aufgenommen und 2 mal mit je 20 ml Wasser, anschließend 2 mal mit je 20 ml einer gesättigten wäßrigen NaCl-Lösung gewaschen. Über MgSO₄ wurde getrocknet und das Solvens im Vakuum entfernt. Der Rückstand wurde an Kieselgel mit EE chromatographiert und man erhielt 90 mg des Produktes (amorph).

| | |
|---|---|
| R_{f}(EE)=0.13 | MS (ES+) : 324 (M+1)⁺ |

### Beispiel 5 N-[2-(4-Fluor-phenoxy)-4-pentafluorsulfanyl-benzoyl]-guanidin

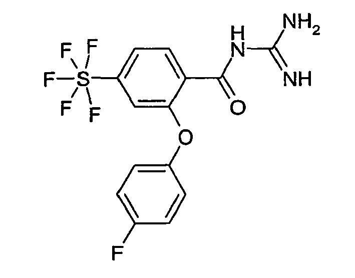

### a) 2-Chlor-4-pentafluorsulfanyl-benzoesäuremethylester

4.3 g 2-Chlor-4-pentafluorsulfanyl-benzoesäure wurden in 50 ml Methanol gelöst und bei RT 5.6 ml SOCl₂ langsam zugetropft. 6 Stunden wurde unter Rückfluss gekocht, die flüchtigen Bestandteile im Vakuum entfernt, der Rückstand mit 100 ml Toluol aufgenommen und erneut die flüchtigen Bestandteile im Vakuum entfernt. Man erhielt 4.1 g eines farblosen Öls.

R_{f} (HEP/DIP 1:1) = 0.44

### b) 2-(4-Fluor-phenoxy)-4-pentafluorsulfanyl-benzoesäuremethylester

300 mg 2-Chlor-4-pentafluorsulfanyl-benzoesäuremethylester, 113 mg 4-Fluorphenol sowie 659 mg Cs₂CO₃ wurden in 1.5 ml DMF (wasserfrei) gelöst und bei 120°C gerührt. Anschließend läßt man abkühlen, verdünnt mit 10 ml EE und wäscht 3 mal mit je 5 ml Wasser. Über MgSO₄ wurde getrocknet und das Solvens im Vakuum entfernt. Man erhielt 120 mg eines amorphen Feststoffs, der an reversed phase Kieselgel chromatographiert wurde:
- Fluss:: 30 ml/min
- Gradient:: ACN=A; Wasser + 0,2% TFA=B 0-3 min 5%A;-14 min 95%A;15-18 min 95%A; -20 min 5%A
- Säule:: XTerra C18 5µm 30x100 mm

Man erhielt 20 mg eines amorphen Feststoffs.

R_{f} Kieselgel (HEP/DIP 1:1) = 0.44

### c) N-[2-(4-Fluor-phenoxy)-4-pentafluorsulfanyl-benzoyl]-guanidin

270 mg KO*t*-Bu sowie 324 mg Guanidin-Hydrochlorid wurden in 1 ml DMF (wasserfrei) bei RT 30 Minuten lang gerührt. Anschließend wurde eine Lösung von 54 mg 2-(4-Fluor-phenoxy)-4-pentafluorsulfanyl-benzoesäuremethylester in 1 ml DMF (wasserfrei) addiert. Danach wurde 2 Stunden lang bei RT gerührt, anschließend auf 10 ml Wasser gegossen, mit wäßriger HCl-Lösung auf pH = 8 eingestellt und 3 mal mit je 5 ml MTB extrahiert. Die organische Phase wurde noch mit 10 ml Wasser gewaschen, dann über MgSO₄ getrocknet und schließlich das Solvens im Vakuum entfernt. Man erhielt 20 mg eines amorphen Feststoffs.

| | |
|---|---|
| R_{f} (EE) = 0.22 | MS (ES+) : 400 (M+1)⁺ |

### Beispiel 6 N-(2-Methyl-5-pentafluorsulfanyl-benzoyl)-guanidin

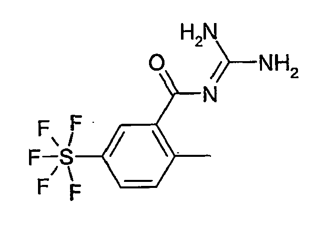

### a) 1-Dichlormethyl-2-nitro-4-pentafluorsulfanyl-benzol

5.4 g KOtBu wurden in 25 ml DMF (wasserfrei) sowie 20 ml THF (wasserfrei) gelöst und auf-73°C gekühlt. Anschließend wurde eine Lösung von 3.0 g 1-Nitro-3-pentafluorsulfanyl-benzol und 1.1 ml CHCl₃ in 15 ml DMF (wasserfrei) so schnell wie möglich (etwa 20 Minuten) zugetropft, wobei die Temperatur unter -67°C gehalten wurde. Eine Minute wurde bei -70°C nachgerührt, dann 15 ml Eisessig in 15 ml Methanol zugespritzt und auf RT erwärmt. Das Reaktionsgemisch wurde auf 200 ml Wasser gegossen und 3 mal mit je 100 ml CH₂Cl₂ extrahiert. Die organische Phase wurde anschließend noch 3 mal mit je 50 ml einer gesättigten wäßrigen Na₂CO₃₋Lösung gewaschen. Über MgSO₄ wurde getrocknet und anschließend das Solvens im Vakuum entfernt. Der Rückstand wurde an Kieselgel chromatographiert mit EE/HEP 1:10 und man erhielt 3.0 g eines farblosen Öls.

R_{f} (EE/HEP 1:10) = 0.78

### b) 2-Methyl-5-pentafluorsulfanyl-anilin

2.0 g 1-Dichlormethyl-2-nitro-4-pentafluorsulfanyl-benzol wurden in 25 ml DME gelöst, 200 mg Pd/C (5%) sowie 100 ml einer gesättigten wäßrigen NaHCO₃-Lösung zugegeben und unter 5 bar H₂ 54 Stunden lang hydriert. Anschließend wurde der Katalysator abfiltriert und die Reaktionslösung 3 mal mit je 50 ml MTB extrahiert. Über MgSO₄ wurde getrocknet und das Solvens im Vakuum entfernt. Man erhielt 1.1 g eines farblosen Öls.

R_{f} (DIP) = 0.42

### c) 2-Brom-1-methyl-4-pentafluorsulfanyl-benzol

1.6 g 2-Methyl-5-pentafluorsulfanyl-anilin wurden in 15 ml Eisessig gelöst und bei 5°C 3 ml einer 48% wäßrigen HBr-Lösung zugetropft. Dabei bildete sich ein Niederschlag. Bei einer Temperatur zwischen 0°C und 5°C wurde dann eine Lösung von 0.52 g NaNO₂ in 3 ml Wasser innerhalb von 10 Minuten zugetropft. Diese Lösung des Diazoniumsalzes wurde anschließend zu einer Suspension von 1.2 g CuBr in 10 ml einer 48% wäßrigen HBr-Lösung und 10 ml Wasser gegossen. 30 Minuten wurde bei RT nachgerührt, dann mit 50 ml Wasser verdünnt und 3 mal mit je 50 ml DIP extrahiert. Die organische Phase wurde noch 2 mal mit je 50 ml einer gesättigten wäßrigen Na₂CO₃-Lösung gewaschen. Über MgSO₄ wurde getrocknet und anschließend das Solvens im Vakuum entfernt. Man erhielt 1.6 g eines leichtbeweglichen Öls.

R_{f} (MTB/n-Pentan 1:5) = 0.67

### d) 2-Methyl-5-pentafluorsulfanyl-benzoesäure

200 mg 2-Brom-1-methyl-4-pentafluorsulfanyl-benzol wurden in 6 ml DMF, 3 ml Tri-n-butylamin und 0.5 ml Wasser gelöst und 129 mg Cs₂CO₃ zugegeben. Dann wurden 15.1 mg Pd(OAc)₂ sowie 35.3 mg Triphenylphosphin zugegeben und unter CO bei Normaldruck 6 Stunden unter Rückfluss gekocht. Das Reaktionsgemisch wurde mit 100 ml EE und 30 ml Wasser verdünnt, mit wäßriger HCl-Lösung auf pH=2-3 eingestellt und 2 mal mit je 30 ml EE extrahiert. Über MgSO₄ wurde getrocknet und anschließend das Solvens im Vakuum entfernt. Chromatographie an reversed phase lieferte 17 mg eines amorphen Feststoffs.
Chromatographiemethoden: 3 HPLC-Läufe; 1 Lauf mit Gradient 1, 2 Läufe mit Gradient 2.
HPLC: Gradient 1 und Gradient 2 beide Laufzeit 20 min
Laufmittel: Wasser (bidest.) + 0,2% TFA, Acetonitril (Chromasolv); Fluss: 30ml/min
Säule: Waters Xterra™ MS C₁₈ 5µm, 30x100mm
MS: Standard 20min, Fraktionierung: nach Zeit

| Gradient 1: | | Gradient 2: | |
|---|---|---|---|
| 0-2,5 min | 10% ACN | 0 - 2,5 min | 10% ACN |
| 3,0 min | 25% ACN | 3,0 min | 20% ACN |
| 14,0 min | 75% ACN | 14,0 min | 60% ACN |
| 15,0 min | 95% ACN | 15,0 min | 95% ACN |
| 17,5 min | 10% ACN | 17,5 min | 10% ACN |

LCMS (ES-) : 261 (M-1)⁻

### e) N-(2-Methyl-5-pentafluorsulfanyl-benzoyl)-guanidin

17.0 mg 2-Methyl-5-pentafluorsulfanyl-benzoesäure wurden in 2 ml DMF (wasserfrei) gelöst, mit 15 mg CDI versetzt und 4 Stunden bei RT gerührt.

Daneben wurden 36.5 mg KOt-Bu und 37.3 mg Guanidin-Hydrochlorid in 2 ml DMF wasserfrei gelöst und 30 Minuten bei RT gerührt. Diese Lösung der Guanidin-Base wurde dann zu obigem lmidazolid zugetropft und 16 Stunden bei RT stehen gelassen. Das Solvens wurde im Vakuum entfernt, der Rückstand mit 5 ml Wasser aufgenommen und mit wäßriger HCl-Lösung auf pH=9 gestellt. Anschließend wurde 3 mal mit je 10 ml EE extrahiert. Über MgSO₄ wurde getrocknet und anschließend das Solvens im Vakuum entfernt. Chromatographie an Kieselgel mit EE lieferte 7.0 mg des Produktes als amorphen Feststoff.

| | |
|---|---|
| R_{f} (EE) = 0.20 | MS(ES+) : 304 (M+1)⁺ |

### Methode NHE-Hemmung

Die NHE-1 Hemmung IC₅₀ [nM] wurde wie folgt bestimmt:
FLIPR-Assay zur Bestimmung von NHE-1 Inhibitoren mittels Messung der pHᵢ₋Erholung in transfizierten Zelllinien, die den humanen NHE-1 exprimieren

Der Assay wird im FLIPR (Fluorescent imaging plate reader) mit schwarzwandigen 96-Well-Mikrotiterplatten mit klarem Boden durchgeführt. Die transfizierten Zelllinien, welche die verschiedenen NHE-Subtypen exprimieren (die parentale Zelllinie LAP-1 weist als Folge von Mutagenese und anschließender Selektion keine endogene NHE-Aktivität auf), werden am Vortag mit einer Dichte von ~25.000 Zellen / Well ausgesät. [Das Wachstumsmedium der transfizierten Zellen (Iscove +10 % fötales Kälberserum) enthält zusätzlich G418 als Selektionsantibiotikum, um die Anwesenheit der transfizierten Sequenzen sicherzustellen.]

Der eigentliche Assay beginnt mit der Entfernung des Wachstumsmediums und Zugabe von 100 µl/Well Beladungspuffer (5 µM BCECF-AM [2',7'-Bis-(Carboxyethyl)-5-(und-6)-Carboxyfluorescein, Acetoxymethyl ester] in 20 mM NH₄Cl, 115 mM Cholinchloride, 1 mM MgCl₂, 1 mM CaCl₂, 5 mM KCI, 20 mM HEPES, 5 mM Glucose; pH 7,4 [mit KOH eingestellt]). Die Zellen werden dann 20 Minuten bei 37°C inkubiert. Diese Inkubation führt zur Beladung der Zellen mit dem fluoreszierenden Farbstoff, dessen Fluoreszenzintensität vom pHi abhängt, und mit NH₄Cl, was zu einer leichten Alkalinisierung der Zellen führt.
[Die nicht fluoreszierende Farbstoff-Vorstufe BCECF-AM ist als Ester membranpermeabel. Intrazellulär wird durch Esterasen der eigentliche Farbstoff BCECF freigesetzt, der nicht membranpermeabel ist.]

Nach dieser 20-minütigen Inkubation wird der Beladungspuffer, der NH₄Cl und freies BCECF-AM enthält, durch dreimaliges Waschen im Zellwasher (Tecan Columbus) mit jeweils 400 µl Waschpuffer (133,8 mM Choline chloride, 4,7 mM KCI, 1,25 mM MgCl₂, 1,25 mM CaCl₂, 0,97 mM K₂HPO₄, 0,23 mM KH₂PO₄, 5 mM HEPES, 5 mM Glucose; pH 7,4 [mit KOH eingestellt]) entfernt. Das in den Wells verbleibende Restvolumen beträgt 90 µl (50 - 125 µl möglich). Dieser Waschschritt entfernt das freie BCECF-AM und führt als Folge der Entfernung der externen NH₄⁺-lonen zu einer intrazellulären Ansäuerung (~ pHi 6.3 - 6.4).

Da das Gleichgewicht von intrazellulärem NH₄⁺ mit NH₃ und H⁺ durch das Entfernen des extrazellulären NH₄⁺ und durch die nachfolgende, augenblicklich ablaufende Passage des NH₃ durch die Zellmembran gestört wird, führt der Waschprozess dazu, das intrazellulär H⁺ zurückbleibt, was die Ursache für die intrazelluläre Ansäuerung ist. Diese kann letztlich zum Zelltod führen, wenn sie lang genug anhält. An dieser Stelle ist es wichtig, dass der Waschpuffer natriumfrei (<1 mM) ist, da extrazelluläre Natrium-Ionen zu einer augenblicklichen Erholung des pHᵢ durch die Aktivität der klonierten NHE-Isoformen führen würde.
Es ist ebenfalls wichtig, dass alle verwendeten Puffer (Beladungspuffer, Waschpuffer, Recoverypuffer) keine HCO₃-lonen enthalten, da die Anwesenheit von Bicarbonat zur Aktivierung störender bicarbonatabhängiger pHᵢ-Regulationssysteme führen würde, die in der parentalen LAP-1 Zelllinie enthalten sind.

Die Mikrotiterplatten mit den angesäuerten Zellen werden dann (bis zu 20 Minuten nach der Ansäuerung) zum FLIPR transferiert. Im FLIPR wird der intrazelluläre Fluoreszenzfarbstoff durch Licht mit einer Wellenlänge von 488 nm, das von einem Argon-Laser erzeugt wird, angeregt, und die Messparameter (Laserleistung, Belichtungszeit und Blende der im FLIPR eingebauten CCD-Kamera) werden so gewählt, dass das durchschnittliche Fluoreszenzsignal pro Well zwischen 30000 und 35000 relativen Fluoreszenzeinheiten liegt.

Die eigentliche Messung im FLIPR beginnt damit, dass Software gesteuert alle zwei Sekunden eine Aufnahme mit der CCD-Kamera gemacht wird. Nach zehn Sekunden wird die Erholung des intrazellulären pHs durch Zugabe von 90 µl Recoverypuffer (133,8 mM NaCl, 4,7 mM KCI, 1,25 mM MgCl₂, 1,25 mM CaCl₂, 0,97 mM K₂HPO₄, 0,23 mM KH₂PO₄, 10 mM HEPES, 5 mM Glucose; pH 7.4 [mit NaOH eingestellt]) mittels des im FLIPR eingebauten 96-Well-Pipettierers eingeleitet.
Als Positivkontrollen (100 % NHE-Aktivität) dienen Wells, denen reiner Recoverypuffer zugegeben wird, Negativkontrollen (0 % NHE-Aktivität) erhalten Waschpuffer. In allen anderen Wells wird Recoverypuffer mit der zweifach konzentrierten Testsubstanz hinzugegeben. Die Messung im FLIPR endet nach 60 Messpunkten (zwei Minuten).

Die Rohdaten werden in das Programm ActivityBase exportiert. Mit diesem Programm werden zunächst die NHE-Aktivitäten für jede getestete Substanzkonzentration und daraus die IC₅₀-Werte für die Substanzen berechnet. Da der Verlauf der pHᵢ-Erholung nicht während des ganzen Experiments linear ist, sondern am Ende aufgrund abnehmender NHE-Aktivität bei höheren pHᵢ-Werten abfällt, ist es wichtig, für die Auswertung der Messung den Teil auszuwählen, in dem die Fluoreszenzzunahme der Positivkontrollen linear ist.

| Beispiel | NHE1-Hemmung IC₅₀ [nM] |
|---|---|
| 1 | 237 |
| 2 | 131 |
| 3 | 14.5 |
| 4 | 220 |
| 5 | 12000 |
| 6 | 20.3 |

## Patentansprüche

1. Pentafluorsulfanyl-benzoylguanidine der Formel I oder II 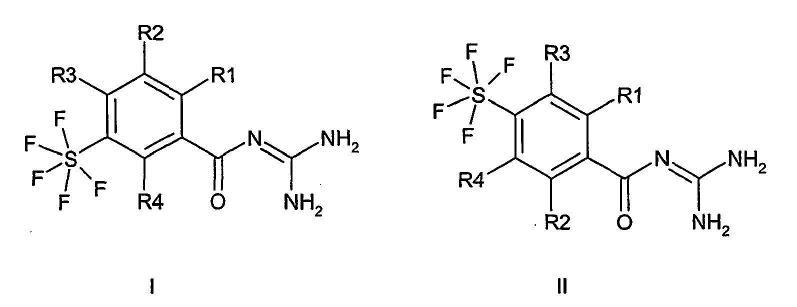 worin bedeuten
R1 Wasserstoff, Alkyl mit 1, 2, 3 oder 4 C-Atomen, Alkoxy mit 1, 2, 3 oder 4 C-Atomen, F, Cl, Br, I, CN, NR10R11, -Oₚ-(CH₂)ₙ-(CF₂)ₒ-CF₃ oder -(SOₘ)_{q}-(CH₂)ᵣ-(CF₂)ₛ-CF₃;
R10 und R11 unabhängig voneinander Wasserstoff, Alkyl mit 1, 2, 3 oder 4 C-Atomen oder ―CH₂-CF₃;
m Null, 1 oder 2
n, o, p, q, r und s unabhängig voneinander Null oder 1;
R2 Wasserstoff, F, Cl, Br, I, -CN, -SO₂CH₃, -(SOₕ)_{z}-(CH₂)ₖ -(CF₂)ₗ-CF₃, Alkyl mit 1, 2, 3, 4, 5 oder 6 C-Atomen, Cycloalkyl mit 3, 4, 5, 6, 7 oder 8 C-Atomen,
in dem 1, 2, 3 oder 4 Wasserstoff-Atome durch Fluoratome ersetzt sein können;
h Null, 1 oder 2;
z Null oder 1;
k Null, 1, 2, 3 oder 4;
I Null oder 1;
oder
R2 -(CH₂)ₜ-Phenyl oder -O-Phenyl,
das unsubstituiert ist oder substituiert ist mit 1, 2 oder 3 Resten ausgewählt aus der Gruppe bestehend aus Reihe F, Cl, Br, I, -Oᵤ-(CH₂)ᵥ-CF₃, Alkoxy mit 1, 2, 3 oder 4 C-Atomen, Alkyl mit 1, 2, 3 oder 4 C-Atomen und -SO₂CH₃;
t Null, 1, 2, 3 oder 4;
u Null oder 1;
v Null, 1, 2 oder 3;
oder
R2 -(CH₂)_{w}-Heteroaryl,
das unsubstituiert ist oder substituiert ist mit 1, 2 oder 3 Resten ausgewählt aus der Gruppe bestehend aus F, Cl, Br, I, -Oₓ-(CH₂)y-CF₃, Alkoxy mit 1, 2, 3 oder 4 C-Atomen und Alkyl mit 1, 2, 3 oder 4 C-Atomen, -SO₂CH₃;
w Null, 1, 2, 3 oder 4;
x Null oder 1;
y Null, 1, 2 oder 3;
R3 und R4 unabhängig voneinander Wasserstoff oder F;
sowie deren pharmazeutisch verträgliche Salze.

2. Verbindung der Formel I oder II nach Anspruch 1, in denen bedeuten:
R1 Wasserstoff, Alkyl mit 1, 2, 3 oder 4 C-Atomen, Alkoxy mit 1, 2, 3 oder 4 C-Atomen, F, Cl, NR10R11, -O-CH₂-CF₃ oder -SOₘ-(CH₂)ᵣ-CF₃;
R10 und R11 unabhängig voneinander Wasserstoff, Alkyl mit 1, 2, 3 oder 4 C-Atomen oder -CH₂-CF₃;
m Null, 1 oder 2;
r Null oder 1;
R2 Wasserstoff, F, Cl, -SO₂CH₃, -(SOₕ)_{z}-(CH₂)ₖ-CF₃, Alkyl mit 1, 2, 3 oder 4 C-Atomen, Cycloalkyl mit 3, 4, 5, 6 oder 7 C-Atomen,
in dem 1, 2, 3 oder 4 Wasserstoff-Atome durch Fluoratome ersetzt sein können;
h Null, 1 oder 2;
z Null oder 1;
k Null, 1, 2, 3 oder 4;
oder
R2 Phenyl oder -O-Phenyl,
das unsubstituiert ist oder substituiert ist mit 1 oder 2 Resten ausgewählt aus der Gruppe bestehend aus F, Cl, -Oᵤ-(CH₂)ᵥ-CF₃, Methoxy, Ethoxy, Alkyl mit 1, 2, 3 oder 4 C-Atomen und -SO₂CH₃;
u Null oder 1;
v Null, 1, 2 oder 3;
oder
R2 Heteroaryl,
das unsubstituiert ist oder substituiert ist mit 1 oder 2 Resten ausgewählt aus der Gruppe bestehend aus F, Cl, -Oₓ-(CH₂)_{y}-CF₃, Methoxy, Ethoxy, Alkyl mit 1, 2, 3 oder 4 C-Atomen und -SO₂CH₃;
x Null oder 1;
y Null, 1, 2 oder 3;
R3 und R4 unabhängig voneinander Wasserstoff oder F; sowie deren pharmazeutisch verträgliche Salze.

3. Verbindung der Formel I oder II nach Anspruch 1 oder 2, in denen bedeuten:
R1 Wasserstoff, Alkyl mit 1, 2, 3 oder 4 C-Atomen, Methoxy, Ethoxy, F, Cl, NR10R11, -O-CH₂-CF₃ oder -SOₘ-(CH₂)ᵣ-CF₃;
R10 und R11 unabhängig voneinander Wasserstoff, Alkyl mit 1, 2, 3 oder 4 C-Atomen oder -CH₂-CF₃;
m Null, 1 oder 2;
r Null oder 1;
R2 Wasserstoff, F, Cl, -SO₂CH₃, -(SOₕ)_{z}-(CH₂)ₖ-CF₃, Alkyl mit 1, 2, 3 oder 4 C-Atomen, Cycloalkyl mit 3, 4, 5, 6 oder 7 C-Atomen,
in dem 1, 2, 3 oder 4 Wasserstoff-Atome durch Fluoratome ersetzt sein können;
h Null oder 2;
z Null oder 1;
k Null oder 1;
oder
R2 Phenyl oder -O-Phenyl,
das unsubstituiert ist oder substituiert ist mit 1 oder 2 Resten ausgewählt aus der Gruppe bestehend aus F, Cl, -O-(CH₂)ᵥ-CF3, Methoxy, Ethoxy, Alkyl mit 1, 2, 3 oder 4 C-Atomen und -SO₂CH₃;
v Null, 1, 2 oder 3;
oder
R2 Heteroaryl,
das unsubstituiert ist oder substituiert ist mit 1 oder 2 Resten ausgewählt aus der Gruppe bestehend aus F, Cl, -O-(CH₂)_{y}-CF₃, Methoxy, Ethoxy, Alkyl mit 1, 2, 3 oder 4 C-Atomen und -SO₂CH₃;
y Null, 1, 2 oder 3;
R3 und R4 Wasserstoff;
sowie deren pharmazeutisch verträgliche Salze.

4. Verfahren zur Herstellung der Verbindung der Formel I oder II oder deren pharmazeutisch verträgliche Salze, **dadurch gekennzeichnet, dass** man eine Verbindung der Formel III oder IV, 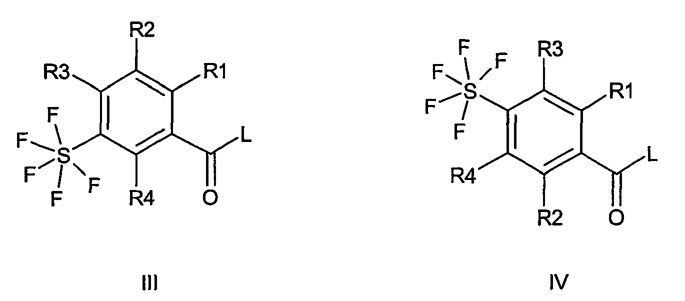 worin R1 bis R4 die in den Ansprüchen 1, 2 und/oder 3 angegebene Bedeutung besitzen und L für eine leicht nucleophil substituierbare Fluchtgruppe steht, mit Guanidin umsetzt.

5. Verbindung der Formel I oder II oder deren pharmazeutisch verträgliche Salze nach einem oder mehreren der Ansprüche 1 bis 3 zu Verwendung als Medikament.

6. Verwendung einer Verbindung der Formel I oder II oder deren pharmazeutisch verträgliche Salze nach einem oder mehreren der Ansprüche 1 bis 3 zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der Prostata-Hyperplasie, der Atherosklerose oder von Störungen des Fettstoffwechsels, des Bluthochdrucks, insbesondere der essentiellen Hypertonie, von Erkrankungen des Zentralnervensystems, insbesondere von Erkrankungen, die durch ZNS-Übererregbarkeit resultieren, wie Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inflammatorischer Erkrankungen, von Erkrankungen, die durch Protozoen verursacht werden, von Malaria und der Hühnercoccidiose und zum Einsatz bei chirurgischen Operationen und Organtransplantationen, zur Konservierung und Lagerung von Transplantaten für chirurgische Maßnahmen, zur Verhinderung altersbedingter Gewebsveränderung, zur Herstellung eines gegen die Alterung gerichteten Medikaments oder zur Lebensverlängerung, zur Behandlung und Senkung der cardiotoxischen Wirkungen in der Thyreotoxikose oder zur Herstellung eines Diagnostikums.

7. Verwendung einer Verbindung der Formel I oder II und/oder deren pharmazeutisch verträgliche Salze nach einem oder mehreren der Ansprüche 1 bis 3 in Kombination mit anderen Arzneimitteln oder Wirkstoffen zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der Prostata-Hyperplasie, der Atherosklerose oder von Störungen des Fettstoffwechsels, des Bluthochdrucks, insbesondere der essentiellen Hypertonie, von Erkrankungen des Zentralnervensystems, insbesondere von Erkrankungen, die durch ZNS-Übererregbarkeit resultieren, wie Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inflammatorischer Erkrankungen, von Erkrankungen, die durch Protozoen verursacht werden, von Malaria und der Hühnercoccidiose und zum Einsatz bei chirurgischen Operationen und Organtransplantationen, zur Konservierung und Lagerung von Transplantaten für chirurgische Maßnahmen, zur Verhinderung altersbedingter Gewebsveränderung, zur Herstellung eines gegen die Alterung gerichteten Medikaments oder zur Lebensverlängerung, zur Behandlung und Senkung der cardiotoxischen Wirkungen in der Thyreotoxikose oder zur Herstellung eines Diagnostikums.

8. Verwendung einer Verbindung der Formel I oder II oder deren pharmazeutisch verträgliche Salze nach Anspruch 7 in der Kombination mit cardiotoxischen und cytotoxischen Arzneimitteln oder Wirkstoffen zur Herstellung eines Medikaments mit verminderten cardiotoxischen und cytotoxischen Eigenschaften.

9. Verwendung einer Verbindung der Formel I oder II oder deren pharmazeutisch verträgliche Salze allein oder in Kombination mit anderen Arzneimitteln oder Wirkstoffen nach Anspruch 6 oder 7 zur Herstellung eines Medikaments zur Behandlung oder Prophylaxe von akuten oder chronischen Schäden, Erkrankungen oder indirekten Folgeerkrankungen von Organen und Geweben, die durch Ischämie- oder durch Reperfusionsereignisse verursacht werden.

10. Verwendung einer Verbindung der Formel I oder II oder deren pharmazeutisch verträgliche Salze allein oder in Kombination mit anderen Arzneimitteln oder Wirkstoffen nach Anspruch 6 oder 7 zur Herstellung eines Medikaments zur Behandlung lebensbedrohlichen Kammerflimmerns des Herzens.

11. Verwendung einer Verbindung der Formel I oder II oder deren pharmazeutisch verträgliche Salze allein oder in Kombination mit anderen Arzneimitteln oder Wirkstoffen nach Anspruch 6 oder 7 zur Herstellung eines Medikaments zur Behandlung oder zur Prophylaxe der Metastasierung.

12. Verwendung einer Verbindung der Formel I oder II oder deren pharmazeutisch verträgliche Salze allein oder in Kombination mit anderen Arzneimitteln oder Wirkstoffen nach Anspruch 6 oder 7 zur Herstellung eines Medikaments zur Behandlung oder Prophylaxe fibrotischer Erkrankungen des Herzens, der Herzinsuffizienz oder des Congestive Heart failure.

13. Heilmittel für die humane, veterinäre oder phytoprotektive Anwendung enthaltend eine wirksame Menge einer Verbindung der Formel I oder II oder deren pharmazeutisch verträgliche Salze nach einem oder mehreren der Ansprüche 1 bis 3, zusammen mit pharmazeutisch annehmbaren Träger- und Zusatzstoffen.

14. Heilmittel für die humane, veterinäre oder phytoprotektive Anwendung enthaltend eine wirksame Menge einer Verbindung der Formel I oder II oder deren pharmazeutisch verträgliche Salze nach einem oder mehreren der Ansprüche 1 bis 3, zusammen mit pharmazeutisch annehmbaren Träger- und Zusatzstoffen in Kombination mit anderen pharmakologischen Wirkstoffen oder Arzneimitteln.

15. Verbindungen der Formel V 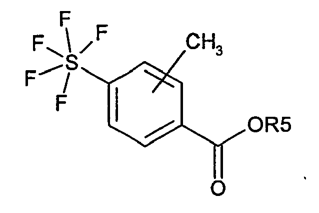 mit R5 gleich Wasserstoff oder (C₁-C₄)- Alkyl.

## Claims

1. A pentafluorosulfanylbenzoylguanidine of the formula I or II 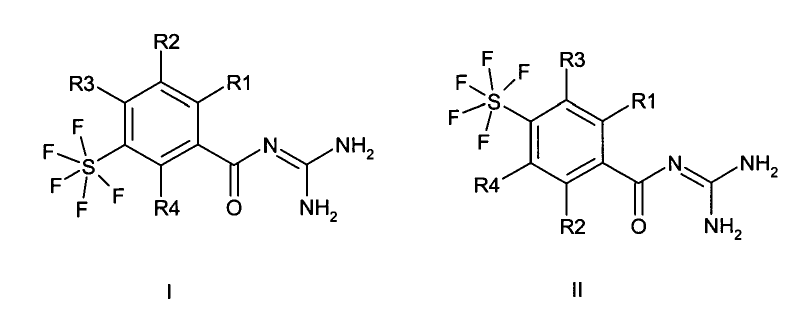 in which
R1 is hydrogen, alkyl having 1, 2, 3 or 4 carbon atoms, alkoxy having 1, 2, 3 or 4 carbon atoms, F, Cl, Br, I, CN, NR10R11, -Op-(CH₂)ₙ-(CF₂)ₒ-CF₃ or -(SOₘ)q-(CH₂)ᵣ-(CF₂)ₛ-CF₃;
R10 and R11 are, independently of one another, hydrogen, alkyl having 1, 2, 3 or 4 carbon atoms or -CH₂-CF₃;
m is zero, 1 or 2
n, o, p, q, r and s are, independently of one another, zero or 1;
R2 is hydrogen, F, Cl, Br, I, -CN, -SO₂CH₃, -(SOₕ)_{z}-(CH₂)ₖ -(CF₂)₁ -CF₃, alkyl having 1, 2, 3, 4, 5 or 6 carbon atoms, cycloalkyl having 3, 4, 5, 6, 7 or 8 carbon atoms,
in which 1, 2, 3 or 4 hydrogen atoms may be replaced by fluorine atoms;
h is zero, 1 or 2;
z is zero or 1;
k is zero, 1, 2, 3 or 4;
I is zero or 1;
or
R2 is -(CH₂)ₜ-phenyl or -O-phenyl,
which is unsubstituted or substituted by 1, 2 or 3 radicals selected from the group consisting of F, Cl, Br, I, -Oᵤ-(CH₂)ᵥ-CF₃, alkoxy having 1, 2, 3 or 4 carbon atoms, alkyl having 1, 2, 3 or 4 carbon atoms and -SO₂CH₃;
t is zero, 1, 2, 3 or 4;
u is zero or 1;
v is zero, 1, 2 or 3;
or
R2 is -(CH₂)_{w}-heteroaryl,
which is unsubstituted or substituted by 1, 2 or 3 radicals selected from the group consisting of F, Cl, Br, I, -Oₓ-(CH₂)_{y}-CF₃, alkoxy having 1, 2, 3 or 4 carbon atoms and alkyl having 1, 2, 3 or 4 carbon atoms, -SO₂CH₃;
w is zero, 1, 2, 3 or 4;
x is zero or 1;
y is zero, 1, 2 or 3;
R3 and R4 are, independently of one another, hydrogen or F;
and the pharmaceutically acceptable salts thereof.

2. A compound of the formula I or II as claimed in claim 1, in which the meanings are:
R1 hydrogen, alkyl having 1, 2, 3 or 4 carbon atoms, alkoxy having 1, 2, 3 or 4 carbon atoms, F, Cl, NR10R11, -O-CH₂-CF₃ or SOₘ- (CH₂)ᵣ-CF₃;
R10 and R11 independently of one another hydrogen, alkyl having 1, 2, 3 or 4 carbon atoms or ―CH₂-CF₃;
m zero, 1 or 2;
r zero or 1;
R2 hydrogen, F, Cl, -SO₂CH₃, -(SOₕ)_{z}-(CH₂)ₖ-CF₃, alkyl having 1, 2, 3 or 4 carbon atoms, cycloalkyl having 3, 4, 5, 6 or 7 carbon atoms,
in which 1, 2, 3 or 4 hydrogen atoms may be replaced by fluorine atoms;
h zero, 1 or 2;
z zero or 1;
k zero, 1, 2, 3 or 4;
or
R2 phenyl or -O-phenyl,
which is unsubstituted or substituted by 1 or 2 radicals selected from the group consisting of F, Cl, -Oᵤ-(CH₂)ᵥ-CF₃, methoxy, ethoxy, alkyl having 1, 2, 3 or 4 carbon atoms and -SO₂CH₃;
u zero or 1;
v zero, 1 2 or 3;
or
R2 heteroaryl,
which is unsubstituted or substituted by 1 or 2 radicals selected from the group consisting of F, Cl, -Oₓ-(CH₂)_{y}-CF₃, methoxy, ethoxy, alkyl having 1, 2, 3 or 4 carbon atoms and -SO₂CH₃;
x zero or 1;
y zero, 1, 2 or 3;
R3 and R4 independently of one another hydrogen or F;
and the pharmaceutically acceptable salts thereof.

3. A compound of the formula I or II as claimed in claim 1 or 2, in which the meanings are:
R1 hydrogen, alkyl having 1, 2, 3 or 4 carbon atoms, methoxy, ethoxy, F, Cl, NR10R11, -O-CH₂-CF₃ or -SOₘ-(CH₂)ᵣ-CF₃;
R10 and R11 independently of one another hydrogen, alkyl having 1, 2, 3 or 4 carbon atoms or -CH₂-CF₃;
m zero, 1 or 2;
r zero or 1;
R2 hydrogen, F, Cl, -SO₂CH₃, -(SOₕ)_{z}-(CH₂)ₖ-CF₃, alkyl having 1, 2, 3 or 4 carbon atoms, cycloalkyl having 3, 4, 5, 6 or 7 carbon atoms,
in which 1, 2, 3 or 4 hydrogen atoms may be replaced by fluorine atoms;
h zero or 2;
z zero or 1;
k zero or 1;
or
R2 phenyl or -O-phenyl,
which is unsubstituted or substituted by 1 or 2 radicals selected from the group consisting of F, Cl, -O-(CH₂)ᵥ-CF₃, methoxy, ethoxy, alkyl having 1, 2, 3 or 4 carbon atoms and -SO₂CH₃;
v zero, 1, 2 or 3;
or
R2 heteroaryl,
which is unsubstituted or substituted by 1 or 2 radicals selected from the group consisting of F, Cl, -O-(CH₂)_{y}-CF₃, methoxy, ethoxy, alkyl having 1, 2, 3 or 4 carbon atoms and -SO₂CH₃;
y zero, 1, 2 or 3;
R3 and R4 hydrogen;
and the pharmaceutically acceptable salts thereof.

4. A process for preparing the compound of the formula I or II or the pharmaceutically acceptable salts thereof, which comprises reacting a compound of the formula III or IV, 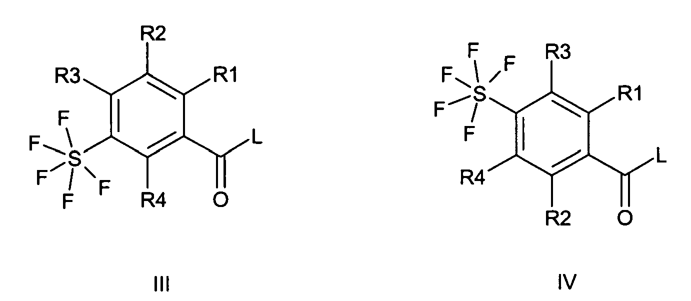 in which R1 to R4 have the meanings stated in claims 1, 2 and/or 3, and L is a leaving group which can readily undergo nucleophilic substitution, with guanidine.

5. A compound of the formula I or II or the pharmaceutically acceptable salts thereof as claimed in one or more of claims 1 to 3 for use as medicament.

6. The use of a compound of the formula I or II or the pharmaceutically acceptable salts thereof as claimed in one or more of claims 1 to 3 for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, in particular essential hypertension, of disorders of the central nervous system, especially of disorders resulting from overexcitability of the CNS, such as epilepsy or centrally induced convulsions, or of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria and of coccidiosis in poultry, and for use for surgical operations and organ transplantations, for preserving and storing transplants for surgical procedures, for preventing age-related tissue change, for producing a medicament directed against aging or for prolonging life, for the treatment and reduction of the cardiotoxic effects in thyrotoxicosis or for producing a diagnostic aid.

7. The use of a compound of the formula I or II and/or the pharmaceutically acceptable salts thereof as claimed in one or more of claims 1 to 3 in combination with other medicaments or active ingredients for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, in particular essential hypertension, of disorders of the central nervous system, especially of disorders resulting from overexcitability of the CNS, such as epilepsy or centrally induced convulsions, or of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria and of coccidiosis in poultry, and for use for surgical operations and organ transplantations, for preserving and storing transplants for surgical procedures, for preventing age-related tissue change, for producing a medicament directed against aging or for prolonging life, for the treatment and reduction of the cardiotoxic effects in thyrotoxicosis or for producing a diagnostic aid.

8. The use of a compound of the formula I or II or the pharmaceutically acceptable salts thereof as claimed in claim 7 in combination with cardiotoxic and cytotoxic medicaments or active ingredients for producing a medicament with reduced cardiotoxic and cytotoxic properties.

9. The use of a compound of the formula I or II or the pharmaceutically acceptable salts thereof alone or in combination with other medicaments or active ingredients as claimed in claim 6 or 7 for producing a medicament for the treatment or prophylaxis of acute or chronic damage, disorders or indirect sequelae of organs and tissues caused by ischemic or reperfusion events.

10. The use of a compound of the formula I or II or the pharmaceutically acceptable salts thereof alone or in combination with other medicaments or active ingredients as claimed in claim 6 or 7 for producing a medicament for the treatment of life-threatening cardiac ventricular fibrillation.

11. The use of a compound of the formula I or II or the pharmaceutically acceptable salts thereof alone or in combination with other medicaments or active ingredients as claimed in claim 6 or 7 for producing a medicament for the treatment or prophylaxis of metastasis.

12. The use of a compound of the formula I or II or the pharmaceutically acceptable salts thereof alone or in combination with other medicaments or active ingredients as claimed in claim 6 or 7 for producing a medicament for the treatment or prophylaxis of fibrotic disorders of the heart, of heart failure or of congestive heart failure.

13. A medicine for human, veterinary or phytoprotective use comprising an effective amount of a compound of the formula I or II or the pharmaceutically acceptable salts thereof as claimed in one or more of claims 1 to 3, together with pharmaceutically acceptable carriers and additives.

14. A medicine for human, veterinary or phytoprotective use comprising an effective amount of a compound of the formula I or II or the pharmaceutically acceptable salts thereof as claimed in one or more of claims 1 to 3, together with pharmaceutically acceptable carriers and additives in combination with other pharmacological active ingredients or medicaments.

15. A compound of the formula V 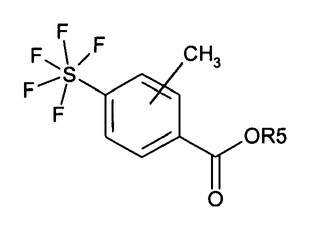 with R5 equal to hydrogen or (C₁-C₄)-alkyl.

## Revendications

1. Pentafluorosulfanyl-benzoylguanidines de formule I ou II 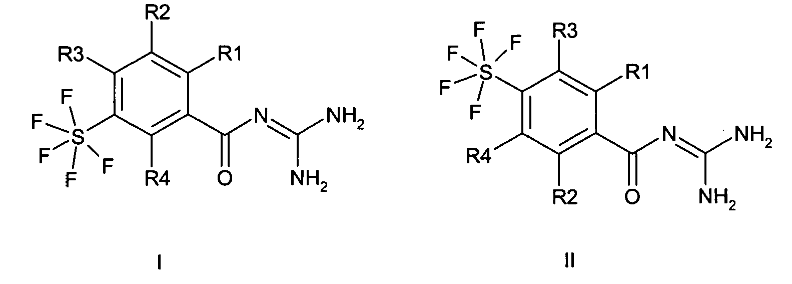 formules dans lesquelles
R1 représente un atome d'hydrogène, un groupe alkyle ayant 1, 2, 3 ou 4 atomes de carbone, alcoxy ayant 1, 2, 3 ou 4 atomes de carbone, F, CI, Br, I, CN, NR10R11, -Oₚ-(CH₂)ₙ-(CF₂)ₒ-CF₃ ou
-(SOₘ)_{q}(CH₂)ᵣ-(CF₂)ₛ-CF₃ ;
R10 et R11 représentent, indépendamment l'un de l'autre, un atome d'hydrogène, un groupe alkyle ayant 1, 2, 3 ou 4 atomes de carbone ou ―CH₂-CF₃ ;
m est zéro, 1 ou 2 ;
n, o, p, q, r et s représentent, indépendamment les uns des autres, zéro ou 1 ;
R2 représente un atome d'hydrogène, F, Cl, Br, I, -CN, -SO₂CH₃, -(SOₕ)_{z}-(CH₂)ₖ-(CF₂)ₗ-CF₃, un groupe alkyle ayant 1, 2, 3, 4, 5 ou 6 atomes de carbone, cycloalkyle ayant 3, 4, 5, 6, 7 ou 8 atomes de carbone, dans lequel 1, 2, 3 ou 4 atomes d'hydrogène peuvent être remplacés par des atomes de fluor ;
h est zéro, 1 ou 2 ;
z est zéro ou 1 ;
k est zéro, 1, 2, 3 ou 4 ;
I est zéro ou 1 ;
ou
R2 représente un groupe -(CH₂)ₜ-phényle ou O-phényle, qui est non substitué ou substitué par 1, 2 ou 3 radicaux choisis dans l'ensemble constitué par la série F, Cl, Br, I, -Oᵤ-(CH₂)ᵥ-CF₃, alcoxy ayant 1, 2, 3 ou 4 atomes, alkyle ayant 1, 2, 3 ou 4 atomes de carbone et ―SO₂CH₃ ;
t est zéro, 1, 2, 3 ou 4 ;
u est zéro ou 1 ;
v est zéro, 1, 2 ou 3 ;
ou
R2 représente un groupe -(CH₂)_{w}-hétéroaryle,
qui est non substitué ou substitué par 1, 2 ou 3 radicaux choisis dans l'ensemble constitué par F, CI, Br, I, -Oₓ(CH₂)_{y}-CF₃, alcoxy ayant 1, 2, 3 ou 4 atomes de carbone et alkyle ayant 1, 2, 3 ou 4 atomes de carbone, ―SO₂CH₃ ;
w est zéro, 1, 2, 3 ou 4 ;
x est zéro ou 1 ;
y est zéro, 1, 2 ou 3 ;
R3 et R4 représentent
indépendamment l'un de l'autre, un atome d'hydrogène ou F ;
ainsi que leurs sels pharmaceutiquement acceptables.

2. Composé de formule I ou II selon la revendication 1, formules dans lesquelles :
R1 représente un atome d'hydrogène, un groupe alkyle ayant 1, 2, 3 ou 4 atomes de carbone, alcoxy ayant 1, 2, 3 ou 4 atomes de carbone, F, CI, NR10R11, -O-CH₂-CF₃ ou -SOₘ-(CH₂)ᵣ-CF₃ ;
R10 et R11 représentent, indépendamment l'un de l'autre, un atome d'hydrogène, un groupe alkyle ayant 1, 2, 3 ou 4 atomes de carbone ou ―CH₂-CF₃ ;
m est zéro, 1 ou 2 ;
r est zéro ou 1 ;
R2 représente un atome d'hydrogène, F, Cl, -SO₂CH₃, -(SOₕ)_{z}-(CH₂)ₖ-CF₃, un groupe alkyle ayant 1, 2, 3 ou 4 atomes de carbone, cycloalkyle ayant 3, 4, 5, 6 ou 7 atomes de carbone,
dans lequel 1, 2, 3 ou 4 atomes d'hydrogène peuvent être remplacés par des atomes de fluor ;
h est zéro, 1 ou 2 ;
z est zéro ou 1 ;
k est zéro, 1, 2, 3 ou 4 ;
ou
R2 représente un groupe phényle ou O-phényle,
qui est non substitué ou substitué par 1 ou 2 radicaux choisis dans l'ensemble constitué par F, CI, -Oᵤ-(CH₂)ᵥ-CF₃, méthoxy, éthoxy, alkyle ayant 1, 2, 3 ou 4 atomes de carbone et -SO₂CH₃ ;
u est zéro ou 1 ;
v est zéro, 1, 2 ou 3 ;
ou
R2 représente un groupe hétéroaryle,
qui est non substitué ou substitué par 1 ou 2 radicaux choisis dans l'ensemble constitué par F, CI, -Oₓ(CH₂)_{y}-CF₃, méthoxy, éthoxy, alkyle ayant 1, 2, 3 ou 4 atomes de carbone et -SO₂CH₃ ;
x est zéro ou 1 ;
y est zéro, 1, 2 ou 3 ;
R3 et R4 représentent indépendamment l'un de l'autre, un atome d'hydrogène ou F ; ainsi que leurs sels pharmaceutiquement acceptables.

3. Composé de formule I ou II selon la revendication 1 ou 2, formules dans lesquelles :
R1 représente un atome d'hydrogène, un groupe alkyle ayant 1, 2, 3 ou 4 atomes de carbone, méthoxy, éthoxy, F, Cl, NR10R11, -O-CH₂-CF₃ ou -SOₘ-(CH₂)ᵣ-CF₃ ;
R10 et R11 représentent,
indépendamment l'un de l'autre, un atome d'hydrogène, un groupe alkyle ayant 1, 2, 3 ou 4 atomes de carbone ou -CH₂-CF₃ ;
m est zéro, 1 ou 2 ;
r est zéro ou 1 ;
R2 représente un atome d'hydrogène, F, CI, -SO₂CH₃, -(SOₕ)_{z}-(CH₂)ₖ-CF₃, un groupe alkyle ayant 1, 2, 3 ou 4 atomes de carbone, cycloalkyle ayant 3, 4, 5, 6 ou 7 atomes de carbone,
dans lequel 1, 2, 3 ou 4 atomes d'hydrogène peuvent être remplacés par des atomes de fluor ;
h est zéro ou 2 ;
z est zéro ou 1 ;
k est zéro ou 1 ;
ou
R2 représente un groupe phényle ou O-phényle,
qui est non substitué ou substitué par 1 ou 2 radicaux choisis dans l'ensemble constitué par F, CI, -O-(CH₂)ᵥ-CF₃, méthoxy, éthoxy, alkyle ayant 1, 2, 3 ou 4 atomes de carbone et -SO₂CH₃ ;
v est zéro, 1, 2 ou 3 ;
ou
R2 représente un groupe hétéroaryle,
qui est non substitué ou substitué par 1 ou 2 radicaux choisis dans l'ensemble constitué par F, Cl, -O-(CH₂)_{y}-CF₃, méthoxy, éthoxy, alkyle ayant 1, 2, 3 ou 4 atomes de carbone et -SO₂CH₃ ;
y est zéro, 1, 2 ou 3 ;
R3 et R4 représentent
un atome d'hydrogène ;
ainsi que leurs sels pharmaceutiquement acceptables.

4. Procédé pour la préparation du composé de formule I ou II ou de ses sels pharmaceutiquement acceptables, **caractérisé en ce qu'**on fait réagir avec de la guanidine un composé de formule III ou IV 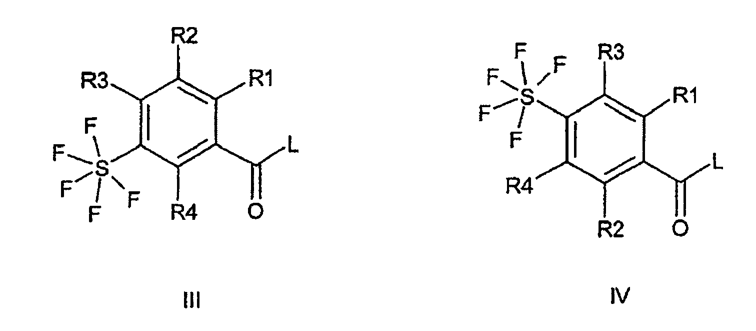 formules dans lesquelles R1 à R4 ont les significations données dans les revendications 1, 2 et/ou 3 et L représente un groupe partant remplaçable par substitution nucléophile.

5. Composé de formule I ou II et/ou sels pharmaceutiquement acceptables d'un tel composé, selon une ou plusieurs des revendications 1 à 3, pour utilisation en tant que médicament.

6. Utilisation d'un composé de formule I ou II ou de ses sels pharmaceutiquement acceptables selon une ou plusieurs des revendications 1 à 3, pour la fabrication d'un médicament destiné au traitement ou à la prophylaxie de troubles, de maladies ou de maladies secondaires indirectes, aigus ou chroniques, d'organes et de tissus, qui sont provoqués par des incidents ischémiques ou par des incidents de reperfusion, au traitement ou à la prophylaxie d'arythmies, de la fibrillation ventriculaire du coeur, mettant la vie en danger, de l'infarctus du myocarde, de l'angine de poitrine, au traitement ou à la prophylaxie d'états ischémiques du coeur, d'états ischémiques du système nerveux périphérique et central ou de l'accident vasculaire cérébral ou d'états ischémiques de tissus et d'organes périphériques, au traitement ou à la prophylaxie d'états de choc, de maladies dans lesquelles la prolifération cellulaire représente une cause primaire ou secondaire, du cancer, de la dissémination de métastases, de l'hypertrophie de la prostate ou de l'hyperplasie de la prostate, de l'athérosclérose ou de troubles du métabolisme lipidique, de l'hypertension artérielle, en particulier de l'hypertonie essentielle, de maladies du système nerveux central, en particulier de maladies qui résultent d'une hyperexcitabilité du système nerveux central, telles que l'épilepsie ou des crises déclenchées par le système nerveux central, de maladies du système nerveux central, en particulier d'états anxieux, de dépressions ou de psychoses, au traitement ou à la prophylaxie du diabète sucré non insulinodépendant (DSNID) ou de complications diabétiques, de thromboses, de maladies faisant suite à un dysfonctionnement endothélial, de la claudication intermittente, au traitement ou à la prophylaxie de maladies fibreuses d'organes internes, de maladies fibreuses du foie, de maladies fibreuses des reins, de maladies fibreuses de vaisseaux et de maladies fibreuses du coeur, au traitement ou à la prophylaxie de l'insuffisance cardiaque ou de la congestive heart failure, de maladies inflammatoires aiguës ou chroniques, de maladies qui sont provoquées par des protozoaires, du paludisme et de la coccidiose du poulet et à l'utilisation dans des interventions chirurgicales et des transplantations d'organes, pour la conservation et le stockage de transplants pour des interventions chirurgicales, à empêcher une altération tissulaire due à l'âge, à la fabrication d'un médicament orienté contre le vieillissement ou pour le prolongement de la vie, au traitement et à l'abaissement des effets cardiotoxiques dans la thyréotoxicose ou à la fabrication d'un agent de diagnostic.

7. Utilisation d'un composé de formule I ou II ou de ses sels pharmaceutiquement acceptables selon une ou plusieurs des revendications 1 à 3, en association avec d'autres médicaments ou principes actifs, pour la fabrication d'un médicament destiné au traitement ou à la prophylaxie de troubles, de maladies ou de maladies secondaires indirectes, aigus ou chroniques, d'organes et de tissus, qui sont provoqués par des incidents ischémiques ou par des incidents de reperfusion, au traitement ou à la prophylaxie d'arythmies, de la fibrillation ventriculaire du coeur, mettant la vie en danger, de l'infarctus du myocarde, de l'angine de poitrine, au traitement ou à la prophylaxie d'états ischémiques du coeur, d'états ischémiques du système nerveux périphérique et central ou de l'accident vasculaire cérébral ou d'états ischémiques de tissus et d'organes périphériques, au traitement ou à la prophylaxie d'états de choc, de maladies dans lesquelles la prolifération cellulaire représente une cause primaire ou secondaire, du cancer, de la dissémination de métastases, de l'hypertrophie de la prostate ou de l'hyperplasie de la prostate, de l'athérosclérose ou de troubles du métabolisme lipidique, de l'hypertension artérielle, en particulier de l'hypertonie essentielle, de maladies du système nerveux central, en particulier de maladies qui résultent d'une hyperexcitabilité du système nerveux central, telles que l'épilepsie ou des crises déclenchées par le système nerveux central, de maladies du système nerveux central, en particulier d'états anxieux, de dépressions ou de psychoses, au traitement ou à la prophylaxie du diabète sucré non insulinodépendant (DSNID) ou de complications diabétiques, de thromboses, de maladies faisant suite à un dysfonctionnement endothélial, de la claudication intermittente, au traitement ou à la prophylaxie de maladies fibreuses d'organes internes, de maladies fibreuses du foie, de maladies fibreuses des reins, de maladies fibreuses de vaisseaux et de maladies fibreuses du coeur, au traitement ou à la prophylaxie de l'insuffisance cardiaque ou de la congestive heart failure, de maladies inflammatoires aiguës ou chroniques, de maladies qui sont provoquées par des protozoaires, du paludisme et de la coccidiose du poulet et à l'utilisation dans des interventions chirurgicales et des transplantations d'organes, pour la conservation et le stockage de transplants pour des interventions chirurgicales, à empêcher une altération tissulaire due à l'âge, à la fabrication d'un médicament orienté contre le vieillissement ou pour le prolongement de la vie, au traitement et à l'abaissement des effets cardiotoxiques dans la thyréotoxicose ou à la fabrication d'un agent de diagnostic.

8. Utilisation d'un composé de formule I ou II ou de ses sels pharmaceutiquement acceptables selon la revendication 7, en association avec des principes actifs ou des médicaments cardiotoxiques et cytotoxiques, pour la fabrication d'un médicament à propriétés cardiotoxiques et cytotoxiques réduites.

9. Utilisation d'un composé de formule I ou II ou de ses sels pharmaceutiquement acceptables, seul(s) ou en association avec d'autres principes actifs ou médicaments selon la revendication 6 ou 7, pour la fabrication d'un médicament destiné au traitement ou à la prophylaxie de troubles, de maladies ou de maladies secondaires indirectes, chroniques ou aigus, d'organes et de tissus, qui sont provoqués par des incidents ischémiques ou par des incidents de reperfusion.

10. Utilisation d'un composé de formule I ou II ou de ses sels pharmaceutiquement acceptables, seul(s) ou en association avec d'autres principes actifs ou médicaments selon la revendication 6 ou 7, pour la fabrication d'un médicament destiné au traitement de la fibrillation ventriculaire du coeur, mettant la vie en danger.

11. Utilisation d'un composé de formule I ou II ou de ses sels pharmaceutiquement acceptables, seul(s) ou en association avec d'autres principes actifs ou médicaments selon la revendication 6 ou 7, pour la fabrication d'un médicament destiné au traitement ou à la prophylaxie de la dissémination de métastases.

12. Utilisation d'un composé de formule I et ou II ou de ses sels pharmaceutiquement acceptables, seul(s) ou en association avec d'autres principes actifs ou médicaments selon la revendication 6 ou 7, pour la fabrication d'un médicament destiné au traitement ou à la prophylaxie de maladies fibreuses du coeur, de l'insuffisance cardiaque ou de la congestive heart failure.

13. Médicament pour l'utilisation humaine, vétérinaire ou phytosanitaire, contenant une quantité efficace d'un composé de formule I ou II ou de ses sels pharmaceutiquement acceptables selon une ou plusieurs des revendications 1 à 3, conjointement avec des véhicules et additifs pharmaceutiquement acceptables.

14. Médicament pour l'utilisation humaine, vétérinaire ou phytosanitaire, contenant une quantité efficace d'au moins un composé de formule I ou II ou de ses sels pharmaceutiquement acceptables selon une ou plusieurs des revendications 1 à 3, conjointement avec des véhicules et additifs pharmaceutiquement acceptables, en association avec d'autres médicaments ou principes actifs pharmacologiques.

15. Composés de formule V 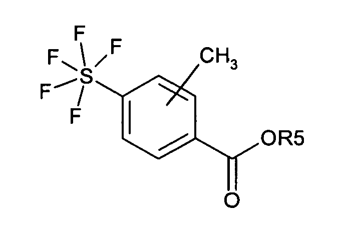 où R5 représente un atome d'hydrogène ou un groupe alkyle en C₁-C₄.
